(19) Europäisches Patentamt
European Patent Office
Office européen des brevets

(11) Publication number: **0 516 859 A1**

(12) **EUROPEAN PATENT APPLICATION published in accordance with Art. 158(3) EPC**

(21) Application number: **91920779.5**

(22) Date of filing: **29.11.91**

(86) International application number: **PCT/JP91/01662**

(87) International publication number: **WO 92/09634 (11.06.92 92/13)**

(51) Int. Cl.[5]: **C07K 15/04**, C12P 21/02, C12N 1/21, C12N 15/51, C12N 15/62, C12N 15/70, G01N 33/576, //(C12P21/02, C12R1:19),(C12N1/21, C12R1:19)

(30) Priority: **29.11.90 JP 325434/90**
**29.11.90 JP 325435/90**
**16.01.91 JP 70231/91**
**19.04.91 JP 179074/91**
**07.06.91 JP 232590/91**

(43) Date of publication of application: **09.12.92 Bulletin 92/50**

(84) Designated Contracting States: **AT BE CH DE DK ES FR GB GR IT LI LU NL SE**

(71) Applicant: **TORAY INDUSTRIES, INC.**
**2, Nihonbashi-Muromachi 2-chome Chuo-ku**
**Tokyo 103(JP)**
Applicant: **Arima, Terukatsu**
**5-1-403, Hirano-cho**
**Kagoshima-shi, Kagoshima 892(JP)**

(72) Inventor: **ARIMA, Terukatsu, 5-1-403, Hirano-cho**
**Kagoshima-shi**
**Kagoshima 892(JP)**
Inventor: **SATO, Atsushi, Toray Industries Residence N-201**
**1-31-17, Tsunishi Kamakura-shi**
**Kanagawa 248(JP)**
Inventor: **IDA, Nobuo, 1111-202, Tebiro**
**Kamakura-shi**
**Kanagawa 248(JP)**
Inventor: **KAZAMI, Jun, Toray Industries Residence L-201**
**2-1-20, Tsunishi Kamakura-shi**
**Kanagawa 248(JP)**

(74) Representative: **Kador & Partner**
**Corneliusstrasse 15**
**W-8000 München 5(DE)**

(54) **NON-A NON-B HEPATITIS VIRUS ANTIGEN PROTEIN.**

(57) Novel non-A non-B hepatitis virus antigen proteins represented by S29 antigen obtained from the serum of a patient with chronic non-A non-B hepatitis. They can be prepared by the genetic recombination technology or chemical synthesis. As the specifically read with the serum of a patient with non-A non-B hepatitis, they can be used in the inspection of such hepatitis.

EP 0 516 859 A1

MSTNPKPQRK TKRNTSRRPQ DVKFPGGGQI
T064
VGGVYLLPRR GPRLGVRATR KTSERSQPRG
T069
RRQPIPKDRR STGKSWGKPG YP

T06A

FIG. 1 RELATIONSHIP BETWEEN AMINO ACID SEQUENCE OF S29 AND DESIGN OF SYNTHETIC PEPTIDES

## Technical Field

The present invention relates to non-A, non-B hepatitis virus-related antigens and method for preparing the same, as well as test agents using the non-A, non-B hepatitis virus-related antigens or substantial equivalents thereof.

The invention is also concerned with non-A, non-B hepatitis virus genome RNA and cDNA for the same.

## Background Art

Hepatitis-causative viruses have been isolated and characterized for hepatitis A which is infected by oral route and for hepatitis B which is infected via blood. As a result, diagnostic methods are established and infection-prophylactic means available presently for both of the hepatitis.

On the other hand, hepatitis that is neither A type nor B type, called non-A, non-B hepatitis, is said to be responsible for about 90% of the post-blood transfusion hepatitis (Nihon Rinsho (Japan Clinicals), vol. 35, page 2724 (1977); Journal of Biological Medicine, vol. 49, page 243 (1976)). According to Koseisho (The Ministry of Health and Welfare, JAPAN), non-A, non-B hepatitis is estimated to be responsible for nearly 40% of the death from hepatic diseases. Most of this type of hepatitis is believed to be ascribed to hepatitis C. Main infective routes are blood transfusion and human blood preparations and would be able to block if an appropriate test method is established. Therefore, development of such test agent is greatly desired.

Recently, Chiron was successful in the cloning of cDNA for HCV (Hepatitis C virus) using as the starting material RNA obtained from chimpanzee serum with non-A, non-B hepatitis caused by injection of serum from non-A, non-B hepatitis patients (O. L. Choo et al., Science, vol. 244, page 359 (1989); European Patent Application No. 88310922.5). On the basis of sequence of the cDNA a variety of antigens have been investigated but up to now none of them could hardly be regarded as effective enough for prophylaxis of HCV infection.

Retrospective studies presented before the symposium "Progress of studies on non-A, non-B hepatitis--Basic and clinical" of the 1990 general conference of the Hepatological Society of Japan revealed that infection could not be prevented in 10 of 14 patients even if the test agents currently used had been developed eight years earlier. This presentation indicated that the test result did not reflect the actual presence of the infectious virus in blood. This is believed to be due to the use of the test agents including antigens which are produced by genetic engineering means from the portion corresponding to non-structural gene products of the viral particles. It may well be said that there are further needs for the development of plural species of antigens in order to carry out reliable HCV tests.

Attempts have also been made by many researchers at cloning of HCV genome, in all of which the PCR technique is employed based upon the base sequence of HCV disclosed by Chiron (Y. Kubota et al., Nucleic Acids Research, vol. 17, page 10367 (1989); H. Okamoto et al., Japan Journal of Experimental Medicine, vol. 60, page 167 (1990) and others).

Because the PCR technique allows cloning of the HCV sequence highly common with the HCV of Chiron only, it is associated with problems to clone HCV genome which is apt to undergo changes of base sequence. Amino acid sequence encoded by a base sequence is also changed with change of the base sequence, and antigenicity of the virus is also variable. Therefore, antibody diagnostic agents using a single antigen would cause problems.

We have now carried out cloning of cDNA based upon the RNA separated from the serum that was derived from plural patients with non-A, non-B hepatitis and found plural species of antigen. Antigenicity of the antigens being individually different, use of the plural different antigens will enable design of highly sensitive antibody test agents in the future.

## Disclosure of the Invention

Thus the invention relates to antigens capable of specifically reacting with serum of non-A, non-B hepatitis patients and a method for preparing the same and agents for detecting a hepatitis related antibody, comprising at least one said specific antigenic protein. The invention is also concerned with cDNA coding for the antigen capable of specifically reacting with serum of non-A, non-B hepatitis patients and sequences of the inherent RNA genome.

## Brief Description of Drawings

Fig. 1 shows relationship between the amino acid sequence of S29 and the design of synthetic peptides.

A:Ala; D:Asp; E:Glu; F:Phe; G:Gly; I:Ile; K:Lys; L:Leu; M:Met; N:Asn; P:Pro; Q:Gln; R:Arg; S:Ser; T:Thr; V:Val; W:Trp; Y:Tyr

Fig. 2 shows results of the ELISA using T06A as an antigen.

Note that Nos. 1 - 21 on the axis of abscissa in the table represent samples from hepatitis C patients, No. 22 a sample from a primary biliary cirrhosis patient, Nos. 23 - 26 samples from hepatitis B patients and Nos. 27 - 30 from normal persons.

Fig. 3 shows results of the ELISA using T064 as an antigen.

Note that Nos. 1 - 21 on the axis of abscissa in the table represent samples from hepatitis C patients, No. 22 a sample from a primary biliary cirrhosis patient, Nos. 23 - 26 samples from hepatitis B patients and Nos. 27 - 30 samples from normal persons.

Fig. 4 shows results of the ELISA using T069 as an antigen.

Note that Nos. 1 - 21 on the axis of abscissa in the table represent samples from hepatitis C patients, No. 22 a sample from a primary biliary cirrhosis patient, Nos. 23 - 26 samples from hepatitis B patients and Nos. 27 - 30 samples from normal persons.

Fig. 5 shows a process for the construction of FUEXS4.

Fig. 6 shows results of the microplate ELISA using T064 and S4 as antigens.

Note that Nos. 1 - 21 on the axis of abscissa in the table represent samples from hepatitis C patients, No. 22 a sample from a primary biliary cirrhosis patient, Nos. 23 - 26 samples from hepatitis B patients, Nos. 27 - 30 samples from normal persons, Nos. 31 and 32 pooled serum of hepatitis C patients, Nos. 33 - 37 N14 antibody (+), C100-3 antibody (+), PCR (+) samples from hepatitis C patients,

Nos. 38 - 42 N14 antibody (+), C100-3 antibody (-), PCR (+) samples, Nos. 43 - 47 N14 antibody (-), C100-3 antibody (+), PCR (+) samples and Nos. 48 - 52 N14 antibody (-), C100-3 antibody (-), PCR (+) samples.

Fig. 7 shows results of the microplate ELISA using T064 and S4 as antigens.

Note that Nos. 1 - 30 represent samples from hepatitis C patients and Nos. 31 - 37 samples from normal persons.

Fig. 8 shows results of the microplate ELISA separately using T064 and S4 as an antigen.

Results of the tests on the reactivity of hepatitis C samples Nos. 1 - 30 separately for T064 and S4.

## Best Mode for Carrying out the Invention

The sequence of cDNA coding for the antigens of the invention was made available as described below.

First, page particles are recovered from serum of plural non-A, non-B hepatitis patients, for example by the polyethylene glycol precipitation method taught by P. Komikuentski et al. in Analytical Biochemistry, vol. 162, page 156 (1987), from which is prepared RNA by means of phenol extraction and isopropanol precipitation.

Synthesis of cDNA from the RNA thus prepared can be effected using a commercially available cDNA synthesis Kit (for example, from BRL) and a random primer (from Takara Shuzo, JAPAN).

Next, the cDNA synthesized can be introduced into EcoR1 site of λ gt11 phage vector using a commercially available λ gt11 cloning kit (from BRL) following by in vitro packaging using a commercially available packaging kit (from Amasham) to prepare a cDNA library.

Since the cDNA is incorporated in $\beta$-gal gene on the λ gt11 phage, its expression is easily detected after infection of E. coli with the phage, as a fused protein with $\beta$-galactosidase by induction of the promoter of lactose operon on said phage with an inducer such as IPTG (isopropyl-$\beta$-D-galactopyranoside).

The λ gt11 phage cDNA libraries are screened for cDNA incorporated and the desired cDNA coding for the antigen capable of specifically reacting with serum of non-A, non-B chronic hepatitis patients can be obtained from the λ gt11 phage cDNA libraries according to the method taught by R. A. Young et al. in Proceeding National Academy of Science U. S. A., vol. 80, page 1194 (1983). Thus infection of E. coli with λ gt11 phage, culture on an IPTG-containing medium, transcription of the plaques formed together with the fused protein with galactosidase expressed on a nitrocellulose membrane, reaction with antibodies contained in the serum derived from non-A, non-B chronic hepatitis patients, and further reaction with anti-human IgG antibodies labeled with HRP (horseradish peroxidase) followed by color development allow selection of the plaque in which a fused protein is reactive with antibodies contained in the serum collected from non-A, non-B chronic hepatitis patients.

The subsequent secondary screening consists of similar selection of the plaque that reacts with antibodies contained in the serum collected from non-A, non-B chronic hepatitis patients but neither with the serum collected from hepatitis B patients nor the serum from normal persons from those selected as above.

Then, the base sequence of cDNA contained in the recombinant λ gt11 phages that have passed the secondary screening is to be determined. The procedures are as follows: First, a small of phage DNA prepared according to the method taught by T. Maniatis et al. in Molecular Cloning, Cold Spring Harbor Lab. (1982), which is then completely or partially fragmented with restriction enzyme EcoR I to give cDNA or cDNA fragments. The cDNA or its fragment is subcloned to a pUC plasmid vector, the plasmid DNA is prepared and the base sequence can be determined, for example, by means of a DNA (Du Pont). SEQ ID NOs. 1 - 12 in Sequence Listing indicate base sequences of the cDNA determined and sequences of the amino acid encoded by the cDNA. SEQ ID NOs. 13 - 24 in Sequence Listing also show sequences of the virus genome RNA conceived from the determined cDNA. In the present invention modified cDNA (that is, in which base sequences are substituted, detected or inserted) may be employed provided that it has substantially the same function as that of the cDNA shown in Sequence Listing. The modification may certainly be to such an extent that the amino acid sequence is different so far as substantially the same function is maintained. It is also possible to use as a functional sequence a complementary sequence of the virus genome RNA sequence (antisense RNA).

Elucidation of cDNA sequence of the virus derived from serum of non-A, non-B chronic hepatitis patients as described above allows for synthesis by means of a peptide synthesizer or expression by means of gene recombination of an amino acid sequence conceived from the sequence of the cDNA obtained to prepare the full length or part of antigen encoded by cDNA for application to serological diagnosis. Thus, the antigen is used for the assay of serum antibody against the antigen obtained, or an antibody against the antigen is in advance prepared and is used for the assay of antigen in serum again the antibody for diagnosis of infections. Note that a monoclonal antibody may be one of the antibodies in advance prepared. In order to confirm the effect of the former method a comparison was made for sensitivity and specificity between the antigens of the invention and known antigens using samples derived from plural patients. Also, synthetic peptides were designed for S29 shown in Sequence Listing in consideration of hydrophilicity and hydrophobicity, and three ELISA's prepared, or which one was used as an antigen.

Serological diagnostic agents using the antigens of the invention will be described below.

First, preparation of the full length or part of the antigen of the invention is by expression in E. coli, yeast, insect cells or animal cells by means of gene recombination technique. As the expression technique is mentioned direct expression of the full length or part of the antigen of the invention by addition to the full length or part of the cDNA coding for the antigen of an initiation signal and a termination signal as needed followed by binding to various known expression vectors.

For example, in E. coli, the cDNA coding for a non-A, non-B specific antigen can be ligated to a sequence known as a promoter such as tryptophan synthetase operon (trp), lactose operon (lac), lambda phage promoter ($P_L$,$P_R$ etc.), chemically synthesized tac promoter (derived from trp and lac) or trc promoter (derived from trp and lac), or additionally, a ribosome binding sequence such as Shine-Dalgarno sequence (SD sequence) or a transcription terminating factor can be added. The ribosome binding sequence may be a consensus sequence prepared, for example, by chemical synthesis, although it may be sequence in E. coli.

The transcription terminating factor, which is not necessarily needed for the expression, is desirably added in order to maintain the plasmid itself stable if the desired protein is highly expressed. As the example is mentioned a terminator for the ribosome RNA gene or trp operon terminator.

The method of expression also includes a method by which a fused protein with another peptide (for example, mouse interferon β, lipocortin, interleukin-2, β-galactosidase or polyhedrin of baculovirus) is expressed, for example, in microorganism or insect cells. Of course, it is also possible to cleave the bond between the subject expression product and another peptide in the fused proteins with a protease (for example, lysine protease: Factor Xa which is a blood coagulation factor) in order to produce the subject antigen as an expression product and subsequently isolate only the antigen portion of the invention for use in the immunoassay.

Transformation by introducing the expression vector thus prepared into a host cell can be carried out using various known techniques, for example, the method disclosed by T. Maniatis et al. in Molecular Cloning, Cold Spring Harbor Lab. (1989). In the case of E. coli, for example, the method described in Molecular Cloning (1989) may also be employed.

Culturing of the transformant may be effected, for example, by using the method described in Molecular Cloning (1989).

The culturing is usually carried out at a temperature of 25 - 42 °C. However, with the promoter shown in Example 8 which induces expression by heat shock, it is desirable to carry out preculturing at approximately 30 °C and expression induction at 42 °C.

Known techniques are used for the purification of the entire or part of the expressed antigen and the preparation of the antigen portion after digestion. Such methods include salt fractionation, ion exchange chromatography, affinity chromatography and centrifugal separation. For example, the methods described in Method in Enzymology (Academic Press, 1980) may be applied. Production and purification of the antigen protein according to the invention is illustrated in Example 8.

In addition, when an epitope portion of the antigen of the invention is revealed it is possible to artificially synthesize a polypeptide containing the epitope portion, which can be used as an antigen either as it is or after binding with another protein. There are known a number of methods for producing such binding, which include use of the disulfide bond (if the peptide has no sulfide group a cysteine residue is added before applying this method) and use of the disulfide/amide bonds. For particulars, see, for example, Seikagaku Jikkenho (Experimental Methods in Biochemistry) 11, Enzyme Immunoassay (Tokyo Kagaku Dojin, JAPAN, 1989).

Next, diagnostic agents for serological diagnosis containing the entire or part of the antigen of the invention as a principal constituent will be described below. A variety of diagnostic agents can be designed in accordance with the immunological detection method employed. For example, in the case where detection is effected by the Ouchterlony method (MO), im munoelectrophoresis (IES, IEP), complement fixation reaction (CF), immune adherence hemoagglutination (IAHA) or single dimension plate immunodiffusion (SRID), the entire or part of the antigen of the invention or the substance containing an artificially synthesized epitope portion as it is may be prepared in an appropriate form. An example may be shown by the use of the single dimension plate immunodiffusion wherein a support selected from materials such as agar, agarose, starch and polyacrylamide is dissolved in a buffer solution to which is then added a substance of the invention followed by mixing, the solution thus obtained is poured onto a glass plate or into a plastic vessel and solidified and wells for the introduction of test serum are provided. Diagnosis can be accomplished by pouring test serum into the well and observing reaction between the diffused antibody and the antigen.

On the other hand, in the case where hemoagglutination reaction (PHA) is employed for detection, it is necessary to bind the full length or part of the antigen of the invention or the substance containing an epitope portion artificially synthesized to microparticles. The preferred microparticles are mammalian or avian blood cells. Such particles may also include approximately 1 - 10μ microparticles in size of polystyrene latex, polyester latex, polyvinyl chloride, bentonite, glass beads, etc. The antigen of the invention and the microparticles may be bound, for example, using glutaradehyde, formaldehyde, tannic acid, bisdiazodized benzidine, chromium chloride or carbodiimide. Diagnosis can be accomplished by mixing test serum with the antigen of the invention immobilized on the microparticles and then observing occurrence of aggregation reaction.

Furthermore, when the antigen of the invention is used for radioimmunoassay (RIA) or enzyme antibody technique (ELISA), it is necessary to bind the full length or part of the antigen of the invention or the substance containing an artificially synthesized epitope portion to an appropriate solid phase as well as to label a secondary antibody to be bound to human antibodies in serum (anti-human antibodies) with a radioactive label or an enzyme. The solid phase that may be used includes a microplate, a tube, beads and magnetic beads, etc. Amount of the antibodies in serum can be detected by the detection of the antibodies bound to the antigen with anti-human antibodies (secondary antibodies). Iodine 125 and iodine 131 may be employed as the radiolabel for the secondary antibodies and can be bound, for example, by means of the chloramine T method. As the labeling enzyme may also be employed, for example, glucose oxidase, alkaline phosphatase, peroxidase or beta galactosidase. Known methods for the binding of the enzyme with antibodies include organic chemical technique, immunological labeling, one via avidin/biotin reaction and combined bringing. For particulars, see, for example, Seikagaku Jikkenho (Experimental Methods in Biochemistry) 11, Enzyme Immunoassay (Tokyo Kagaku Dojin, JAPAN, 1989). Furthermore, it is also possible to use a special enzyme antibody method (ELISA) in which antibodies are detected by catching the antibodies in sample serum on the solid phase bound with the antigen of the invention and further reacting the antibody complexes with an enzyme-labeled antigen. An example of the enzyme antibody method using a microplate according to the invention is given in Example 9.

In the present invention a part of the antigen proteins on Sequence Listing (SEQ ID NOs. 1 - 12) may also be employed as equivalent.

In some of the serological diagnostic methods as described above, it is also preferred from a view point of sensitivity to use a combination of the antigens of the invention on Sequence Listing (SEQ ID NOs. 1 - 12) or the epitope sites thereof. In addition, the techniques known as competition method or sandwich method may also be applied, and various labelings including fluorescent labeling may also be used. In addition to the use as an immunological test agent, the test agents of the invention may also be utilized as a DNA probe.

The invention will be described in more detail below with reference to examples.

Example 1

Preparation of virus RAN

To 1.8 L of C-100-3 antibody (Chiron) positive serum from non-A, non-B chronic hepatitis patients was added 72 g of polyethylene glycol 4000 (Nakarai Kagaku, JAPAN), and the mixture allowed to stand in ice for one hour to precipitate the virus. A viral fraction was obtained by centrifugal separation at 4°C and 5 Krpm for 20 min. followed by addition of 100 ml of a denaturing solution (6 M guanidine thiocyanate, 37.5 mM sodium citrate pH 7.0, 0.75% Sarcosyl, 1.05 ml 2-mercaptoethanol), and the mixture immediately shaken. Subsequently, 15 ml of sodium acetate (pH 4.0), 150 ml of phenol (saturated with distilled water) and 30 ml of chloroform/isoamyl alcohol (49 : 1) were added in this order, and the mixture blended. After centrifuged the aqueous layer was transferred to another vessel and mixed with an equal volume of isopropyl alcohol, and the mixture allowed to stand at -20 °C for 2 - 3 days to precipitate RNA. After again centrifuged at room temperature and 15 Krpm for 20 min. precipitates were dissolved in 1 ml of the denaturing solution. To the solution was added 1 ml of isopropanol, and the mixture storage at -70 °C. There was prepared approximately 10 $\mu$g of RNA.

Example 2

Preparation of a cDNA library

The RNA stock solution was centrifuged, and pellets then washed with 70% ethanol and dissolved in 20 $\mu$l of diethyl pyrocarbonate-treated sterilized water. The RNA was determined by DNA DUIP STICK (Invitrogen).

Starting with approximately 0.5 $\mu$g of the RNA cDNA was synthesized by the use of a cDNA synthesis system and a cDNA cloning system (BRL) and cloned to $\lambda$ gt11. In this cDNA synthesis a random primer (Takara Shuzo, JAPAN) was used in place of the oligo dT primer since presence of the ploy A chain was not clear in this viral RNA.

Then, the recombinant $\lambda$ gt11 was subjected to packaging by the use of a $\lambda$ DNA packaging kit (Amasham), and plaques were formed on an indicator strain Y1090. The medium used for the formation of plaques consisted of a lower layer of 1.5% agar L medium (1% bactotryptone, 0.5% yeast extract, 1% sodium chloride) and an upper layer of 0.7% agarose L medium. A cDNA library with approximately 5 x $10^6$ pfu was prepared.

Example 3

Primary screening

The cDNA library constructed above was dispersed on a plate 150 mm in diameter (Vecton Dickinson) so as to develop approximately 1.0 x $10^4$ pfu, and the plate cultured at 43°C for 3 - 4 hours to form plaques. Then, a nitrocellulose filter (Hibond C Extra, Amasham) which had been immersed in 10 mM IPTG (isopropyl-$\beta$-D-thiogalactopyranoside) and air dried was placed on the plate and cultured at 37°C for 3 hours. This results in expression of amino acid sequences encoded by the cloned cDNA. The antigens plotted on the filter were reacted with an approximately 25 dilution of pooled serum (pretreated with E. coli) from several non-A, non-B hepatitis patients as the primary antibody and peroxidase-labeled anti-human IgG (Kappel) as the secondary antibody. Color development indicated positive clone. There were obtained 56 positive clones in total. The filter and the plate were then adjusted in place, and the agar at the positions which were likely to correspond to the positive clone was picked up with a sterilized toothpick and suspended in 1 ml of SM buffer (5.8 g NaCl, 2 g $MgSO_4.7H_2O$, 6.05 g tris base, 5 ml 2% gelatin/L $H_2O$). The suspension with 10 $\mu$l of chloroform added was stored at 4 °C as a phage solution.

Example 4

Secondary screening and specificity test

The phage solutions obtained from the positive clones were appropriately diluted with SM buffer and then dispersed on a plate so as to form approximately 300 plaques per plate. Positive clones were color-developed in the same manner as in Example 3. The positive clone was picked up with a sterilized toothpick and suspended in 0.5 ml of SM buffer. The suspension with 10 μml of chloroform added was stored at 4 ° C for use as a single plaque phage solution.

Reactivity was examined for the clones that were positive in the secondary screening with two serum samples from normal persons (negative with C100-3 antibody, Chiron) and one serum sample from a hepatitis B patient. The positive clone was mixed with λ gt11 (vector without cDNA sequence) at a plaque number ratio of 1 : 5, and plaques were formed on an indicator strain Y1090. Subsequently, immunoplaque assay was carried out in the same manner as in Example 3 to see whether it is negative or positive.

## Example 5

### Determination of the base sequence of cDNA

Phage DNA was extracted by the following procedures.

A mixture of 10 μl of each of the phage solutions with 0.5 ml of a culture of Y1090 was allowed to stand at room temperature for 15 min. to cause infection with the phage. To the mixture was added 5 ml of L medium containing 5 mM calcium chloride and 50 μg/ml ampicillin followed by incubation at 43 ° C for 4 hours. The culture was centrifuged, and to the supernatant was added an equal amount of a solution (20% polyethylene glycol 6000, 50 mM tris hydrochloride (pH 7.5), 2M sodium chloride, 4 mM magnesium sulfate, 2% gelatin). The mixture was allowed to stand in ice for one hour and centrifuged to precipitate the phage. The pellets were suspended in 0.75 ml of L medium, to which was then added an equal volume of a suspension of DE 52 in L medium. The mixture was centrifuged, and to the supernatant were added 17.5 μl of 0.1 mg/ml protease K and 42.5 μl of a 10% SDS solution to destroy the phage particles. The phage DNA was recovered by precipitation with isopropanol and dissolved in 40 μl of TE buffer.

A DNA with 30 bases containing EcoR I site λ gt11 shown below was synthesized by means of a DNA synthesizer (Applied Biosystem). A PCR reaction was applied to the DNA obtained above using the synthetic DNA as a primer. The amplified DNA product was confirmed by means of the agarose gel electrophoresis (4% NUCLEVE GTG agarose, Takara Shuzo, JAPAN). At the same time the DNA thus produced was cleaved with a restriction enzyme EcoR I, and the cDNA portion cloned at the EcoR I site of pUC18.

A small amount of pUC18 DNA having the cDNA sequence was prepared by the alkali SDS method (e. g. T. Maniatis et al., Molecular Cloning, Cold Spring Harbor Lab. (1989)), and the cDNA then sequenced by means of a DNA sequencer (Du Pont).

## Example 6

### Evaluation of the antigens produced

Nine clones of the 30 clones for which the base sequence was determined were reacted by the method shown in Example 3 with serum samples from non-A, non-B chronic hepatitis patients, hepatitis B patients, primary biliary cirrhosis patients and normal persons. Results of the reaction were compared with those obtained by a prior art technique. The results are shown in Table 1. The prior art technique and known clone used were Ortho HCV-Ab ELISA Test (Ortho Diagnostic Systems) and Arima N14 clone (Mevio 1990, vol. 7, No. 1, p. 20, Medical View, JAPAN), respectively.

In Table 1 clone S29 has a base sequence corresponding to the antigen of SEQ ID NO. 1 and clone S4 to the antigen of SEQ ID NO. 2. Additionally, clone S12 corresponds to SEQ ID NO. 4, S41 to SEQ ID NO. 9, S34 to SEQ ID NO. 7, S13 to SEQ ID NO. 5, S31 to SEQ ID NO. 6, S39 to SEQ ID NO. 8 and S1 to SEQ ID NO.3.

CH: chronic hepatitis patient; LC: cirrhosis patient; AH: hepatitis patient; PBC: primary biliary cirrhosis patient; NORMAL: normal person; Nos. 23 - 26: hepatitis B patient; HBsAg: HBs antigen; +: positive; -: negative.

(TABLE 1)

EVALUATION OF HCV CLONES BY INDICATOR SERUM

| SAMPLE No. | DIAGNOSIS | TRANSFUSION | HBsAg | ARIMA N14 | CHIRON c100-3 | TORAY CLONE S29 | S4 | S12 | S41 | S34 | S13 | S31 | A39 | S1 |
|---|---|---|---|---|---|---|---|---|---|---|---|---|---|---|
| 1 | CH | + | - | + | + | + | + | + | + | + | + | + | - | - |
| 2 | CH | + | - | + | + | + | - | - | - | + | + | + | + | + |
| 3 | CH | + | - | + | + | + | + | + | + | + | - | + | + | + |
| 4 | CH | + | - | + | - | + | - | - | - | - | - | + | - | - |
| 5 | CH | + | - | + | + | + | + | + | + | + | - | + | + | + |
| 6 | CH | + | - | + | + | - | + | + | - | - | - | - | - | - |
| 7 | CH | + | - | - | + | + | + | + | + | - | - | + | + | - |
| 8 | CH | + | - | - | + | + | + | + | + | - | - | + | - | - |
| 9 | LC | + | - | + | + | + | - | - | - | + | + | + | - | + |
| 10 | CH | - | - | - | - | + | - | - | + | + | - | + | + | + |
| 11 | CH | - | - | + | + | + | + | + | + | + | - | + | + | + |
| 12 | CH | - | - | + | + | + | + | + | + | + | - | + | + | + |
| 13 | CH | - | - | - | - | + | - | - | - | + | - | + | - | - |
| 14 | LC | - | - | + | + | + | + | + | + | + | - | + | + | + |
| 15 | CH | - | - | - | + | - | + | - | - | - | - | - | - | - |
| 16 | LC | - | - | + | + | + | + | + | + | - | - | + | - | - |
| 17 | LC | - | - | + | + | + | + | - | + | - | + | + | + | - |
| 18 | LC | - | - | + | + | + | - | - | + | + | + | + | - | + |
| 19 | LC | - | - | + | - | + | + | - | - | - | - | + | - | - |
| 20 | AH | - | - | - | - | - | - | - | - | - | - | - | - | - |
| 21 | AH | - | - | + | - | + | - | - | - | - | - | + | + | + |
| 22 | PBC | + | - | - | - | - | - | - | - | - | - | - | - | - |
| 23 | CH | - | + | - | - | - | - | - | - | - | - | - | - | - |
| 24 | CH | - | + | - | - | - | - | - | - | - | - | - | - | - |
| 25 | CH | - | + | - | - | - | - | - | - | - | - | - | - | - |
| 26 | CH | - | + | - | - | - | - | - | - | - | - | - | - | - |
| 27 | NORMAL | - | - | - | - | - | - | - | - | - | - | - | - | - |
| 28 | NORMAL | - | - | - | - | - | - | - | - | - | - | - | - | - |
| 29 | NORMAL | - | - | - | - | - | - | - | - | - | - | - | - | - |
| 30 | NORMAL | - | - | - | - | - | - | - | - | - | - | - | - | - |

## Example 7

### Examination of the epitope region of S29

Synthetic peptides were designed for the clone S29 which produced good results in Example 6 in due consideration of hydrophilicity, and an ELISA system was prepared. First, peptides with around 30mers were synthesized for regions of higher hydrophilicity (Fig. 1). The synthesized peptides were immobilized on a 96-well microplate, to which were then added 100 µl of a buffer solution and 10 µl of a serum sample. The mixture was allowed to react at 37 °C for 60 min. After washed, the plate was subjected to reaction at 37 °C for 15 min. With peroxidase-labeled gout anti-human IgG antibodies (Zymed Laboratories) for the T069 system as well as for the T06A system, and with peroxidase-labeled mouse anti-human IgG antibodies (manufactured by Zymed) for the T064 system. After additionally washed the plate was subjected to addition of a substrate solution and reaction at 37 °C for 15 min. followed by termination of the reaction by addition of 1 N sulfuric acid. Absorbance at 450 nm was then measured. The results are shown in Figs. 2, 3 and 4. All of the three peptides reacted specifically with hepatitis C serum, among which T064 exerted the best reactivity.

Example 8

Expression of S4 antigen in E. coli

An antigen was prepared for the clone S4 which gave good results in Example 6. S4 gene was inserted so as to contain a recognition site for factor Xa on the β-galactosidase C terminal side of plasmid pUEX1 having λ phage cI857, promoter and coding for β-galactosidase (Amasham) (Fig. 5). Since a factor Xa site is present between β-galactosidase and S4, there can be obtained S4 containing no protective protein by digestion with factor Xa after protein expression.

First, a plasmid was prepared in which Kpn 1 linker (5-GGGTACCC-3, Takara Shuzo, JAPAN) was ligated to Sma 1 site present on the β-galactosidase C terminal side (pU EXK-1).

A S4 fragment containing factor Xa-recognizing site was ligated to Kpn I, BamH I site of pUEXK-1 by separately preparing a DNA fragment in which a cDNA fragment of S4 was incorporated in EcoR I site and utilizing the Kpn I, BamH I site (pUEXS4).

The pUEXS4 thus prepared was transformed into E. coli HB 101, and the resulting strain inoculated on 5 ml of LB medium (trypton 1%, yeast extract 0.5%, NaCl 0.5%, ampicillin 100 µg/ml) and preincubated overnight at 30 °C. After the overnight incubation, 200 µl of the culture was inoculated on 10 ml of LB medium containing ampicillin and subjected to shaking culture at 30 °C for 2 hours. The culture temperature was then raised to 42 °C, and shaking culture continued for an additional 2 hours. Protein expression was confirmed using SDS-PAGE for 1 ml of the culture after centrifugation, collection of the cells and lysis by addition of 1% SDS and 2-mercaptoethanol. The fused protein of S4 and galactosidase was observed specifically when cultured at 42 °C, and the protein amounted to 15 -20% of the whole protein. The fused protein, which formed insoluble inclusion bodies, was washed with a guanidine solution or 4 M urea to remove soluble proteins. It was then solubilized with 8 M urea and dialyzed against factor Xa buffer (20 mM tris-HCl pH 8, 100mM NaCl, 2 mM CaCl). Antigenicity of the fused protein of S4 and galactosidase was examined for reactivity with serum from hepatitis C patients after electrophoresis, transcription of the protein to an nitrocellulose filter. It was confirmed that the expressed protein did not react with serum from normal persons but specifically with serum from hepatitis C patients. Furthermore, S4 protein (23 Kd) obtained by digestion with factor Xa was also examined for reactivity with serum from hepatitis C patients to confirm that the expressed protein did not react with serum from normal persons but specifically with serum from hepatitis C patients. The purified peptide was also examined for N-terminal amino acid sequence to confirm that it is in conformity with the sequence conceived from the gene sequence.

Example 9

Preparation of microplate EIA with combination of S29 and S4 antigens

The recombinant antigen S4 described in Example 8 was further purified , and microplate EIA prepared using the synthetic peptide T064 described in Example 7 and the S4 as antigens. The synthesized peptide T064 and the purified recombinant antigen S4 were immobilized on a 96-well microplate, to which were then added 100 µl of a buffer solution and 10 µl of a serum sample followed by reaction at 37 °C for 60 min. The plate was washed and subsequently subjected to reaction at 37°C for 15 min. with peroxidase-labeled mouse anti-human IgG monoclonal antibody (Zymed Laboratories). To the plate after further washed was added a substrate solution, and the mixture at 37 °C for 15 min. The reaction was terminated by addition of 1 N sulfuric acid followed by measurement of absorbance at 450 nm. The results are shown in Figs. 6, 7 and 8. The reaction was judged positive in 70 of 71 serum samples from hepatitis C patients but was negative in 4 samples of hepatitis B, 11 samples of normal persons and one sample of primary biliary cirrhosis. Further, reactivity was examined with S4 and S29 antigens, respectively, for 30 samples of the 71 samples from hepatitis C. It was found that 22 samples were positive for both S4 and S29, 3 samples for S4 only, 4 samples for S29 only and one sample for none of the two. Thus, difference in reactivity of serum samples between S29 and S4 antigens was demonstrated, and usefulness of the two-antigen combination was confirmed.

Industrial Applicability

According to the present invention there is provided cDNA sequence coding for novel antigens that are reactive with serum from non-A, non-B chronic hepatitis patients. The cDNA of the present invention is shown to be applicable in the development of immunological diagnostic agents. Furthermore, ELISA using the S29-derived synthetic peptide T064 and recombinant antigens obtained hereby is demonstrated to be highly sensitive to serum from non-A, non-B hepatitis patients and useful, for example, in blood screening.

SEQ ID NO: 1
SEQUENCE LENGTH: 263
SEQUENCE TYPE: nucleic acid
STRANDEDNESS: double
TOPOLOGY: linear
MOLECULE TYPE: cDNA to genomic RNA
ORIGINAL SOURCE: non-A, non-B hepatitis virus
SEQUENCE DESCRIPTION:

```
                                                   CTCGTAGACCGTGCATC   17

ATG AGC ACA AAT CCT AAA CCT CAA AGA AAA ACC AAA AGA AAC ACA AGC    65
Met Ser Thr Asn Pro Lys Pro Gln Arg Lys Thr Lys Arg Asn Thr Ser
  1               5                  10                  15

CGC CGC CCA CAG GAC GTC AAG TTC CCG GGT GGC GGT CAG ATC GTT GGC   113
Arg Arg Pro Gln Asp Val Lys Phe Pro Gly Gly Gly Gln Ile Val Gly
             20                  25                  30

GGA GTT TAC TTG CTG CCG CGC AGG GGC CCC AGG TTG GGT GTG CGC GCG   161
Gly Val Tyr Leu Leu Pro Arg Arg Gly Pro Arg Leu Gly Val Arg Ala
         35                  40                  45

ACA AGG AAG ACT TCC GAG CGA TCC CAG CCG CGT GGG AGA CGC CAG CCC   209
Thr Arg Lys Thr Ser Glu Arg Ser Gln Pro Arg Gly Arg Arg Gln Pro
     50                  55                  60
```

ATC CCT AAA GAT CGG CGC TCC ACC GGC AAG TCC TGG GGA AAG CCA GGA 257
Ile Pro Lys Asp Arg Arg Ser Thr Gly Lys Ser Trp Gly Lys Pro Gly
65 70 75 80

TAT CCT 263
Tyr Pro

SEQ ID NO: 2
SEQUENCE LENGTH: 651
SEQUENCE TYPE: nucleic acid
STRANDEDNESS: double
TOPOLOGY: linear
MOLECULE TYPE: cDNA to genomic RNA
ORIGINAL SOURCE: non-A, non-B hepatitis virus
SEQUENCE DESCRIPTION:

CT 2

ACG GGC GCC CCC ATT ACG TAC TCC ACC TAT GGT AAG TTC CTT GCC GAC 50
Thr Gly Ala Pro Ile Thr Tyr Ser Thr Tyr Gly Lys Phe Leu Ala Asp
1 5 10 15

GGT GGT TGC TCT GGG GGC GCC TAT GAC ATC ATA ATG TGC GAT GAG TGC 98
Gly Gly Cys Ser Gly Gly Ala Tyr Asp Ile Ile Met Cys Asp Glu Cys
20 25 30

CAC TCA ACT GAC TCG ACC TCC ATC TTG GGC ATT GGC ACG GTC CTG GAC 146
His Ser Thr Asp Ser Thr Ser Ile Leu Gly Ile Gly Thr Val Leu Asp
35 40 45

```
CAA GCG GAG ACG GCT GGA GCG CGA CTT GTC GTG CTC GCC ACC GCT ACC      194
Gln Ala Glu Thr Ala Gly Ala Arg Leu Val Val Leu Ala Thr Ala Thr

        50                  55                  60
CCT CCG GGA TCG GTC ACT GTA CCA CAT CCC AAT ATC GAG GAG GTG GCC      242
Pro Pro Gly Ser Val Thr Val Pro His Pro Asn Ile Glu Glu Val Ala
65                  70                  75                  80

CTG TCC AAC ACT GGA GAG ATT CCC TTC TAT GGC AAA GCC ATC CCT ATC      290
Leu Ser Asn Thr Gly Glu Ile Pro Phe Tyr Gly Lys Ala Ile Pro Ile
                85                  90                  95

GAG ACC ATC AAG GGG GGG AGG CAT CTC ATT TTT TGC CAC TCT AAG AAG      338
Glu Thr Ile Lys Gly Gly Arg His Leu Ile Phe Cys His Ser Lys Lys
            100                 105                 110

AAG TGT GAT GAG CTC GCC ACA AAG CTG TCG GCC CTC GGA CTC AAT GCT      386
Lys Cys Asp Glu Leu Ala Thr Lys Leu Ser Ala Leu Gly Leu Asn Ala
        115                 120                 125

GTA GCG TAC TAC CGG GGC CTT GAT GTG TCC GTT ATA CCA ACA AGC GGA      434
Val Ala Tyr Tyr Arg Gly Leu Asp Val Ser Val Ile Pro Thr Ser Gly
    130                 135                 140

GAC GTC GTT GTC GTG GCA ACA GAC GCT CTA ATG ACG GGC TAC ACC GGT      482
Asp Val Val Val Val Ala Thr Asp Ala Leu Met Thr Gly Tyr Thr Gly
145                 150                 155                 160
```

GAC TTT GAC TCA GTG ATC GAC TGT AAT ACA TGC GTC ACC CAG ACA GTC 530
Asp Phe Asp Ser Val Ile Asp Cys Asn Thr Cys Val Thr Gln Thr Val
165 170 175

GAT TTC AGC TTG GAC CCT ACC TTC ACC ATT GAG ACG ACA ACC GTG CCT 578
Asp Phe Ser Leu Asp Pro Thr Phe Thr Ile Glu Thr Thr Thr Val Pro
180 185 190

CAA GAC GCG GTG TCA CGC TCG CAG CGG CGA GGT AGG ACT GGT AGG GGC 626
Gln Asp Ala Val Ser Arg Ser Gln Arg Arg Gly Arg Thr Gly Arg Gly
195 200 205

AGG GGG GGC ATA TAC AGG TTT GTA G 651
Arg Gly Gly Ile Tyr Arg Phe Val
210 215

SEQ ID NO: 3
SEQUENCE LENGTH: 561
SEQUENCE TYPE: nucleic acid
STRANDEDNESS: double
TOPOLOGY: linear
MOLECULE TYPE: cDNA to genomic RNA
ORIGINAL SOURCE: non-A, non-B hepatitis virus
SEQUENCE DESCRIPTION:

```
GAA TTC CAT TAC CTA CCG CCT ACT GTT GCA GGC TGT GCT CTC CCC CCC    48
Glu Phe His Tyr Leu Pro Pro Thr Val Ala Gly Cys Ala Leu Pro Pro
  1               5                  10                  15

CCC AAG AAG ACC CCG ACG CCC CCC CCG AGG AGG CGC CGG ACA GTG GCC    96
Pro Lys Lys Thr Pro Thr Pro Pro Pro Arg Arg Arg Arg Thr Val Ala
             20                  25                  30

TTG GAC GGG AGC ACC ATT GGA GAC GTC CTC CAG CAG TTG GCC GTC AAG   144
Leu Asp Gly Ser Thr Ile Gly Asp Val Leu Gln Gln Leu Ala Val Lys
         35                  40                  45

ACC TTC GGC CAG CCC CCC CCG AGC GGC GAC TCG GGC CCC TCC ACG GGG   192
Thr Phe Gly Gln Pro Pro Pro Ser Gly Asp Ser Gly Pro Ser Thr Gly
     50                  55                  60

GCG GAC GTC GTC GGC TCT GGT GGT CGG ACG CCC CCT GAT GAA TTG GCT   240
Ala Asp Val Val Gly Ser Gly Gly Arg Thr Pro Pro Asp Glu Leu Ala
 65                  70                  75                  80
```

CTC TCG GAG ACA GGT TCT GTC TCT TCC ATG CCC CCG CTC GAG GGG GAG 288
Leu Ser Glu Thr Gly Ser Val Ser Ser Met Pro Pro Leu Glu Gly Glu
85 90 95

CCT GGG GAT CCG GAC CTA GAG CCT GAG CGG GTA GAG CTT CAG CCC CCC 336
Pro Gly Asp Pro Asp Leu Glu Pro Glu Arg Val Glu Leu Gln Pro Pro
100 105 110

CCC CAG GGG GGG GAG GAA GCT CCC GGC TCG GAC TCG GGG TCC TGG TCT 384
Pro Gln Gly Gly Glu Glu Ala Pro Gly Ser Asp Ser Gly Ser Trp Ser
115 120 125

ACT TGC TCC GAG GAG AGT GAC TCC GTC GTG TGC TGC TCC ATG TCT TAC 432
Thr Cys Ser Glu Glu Ser Asp Ser Val Val Cys Cys Ser Met Ser Tyr
130 135 140

TCC TGG ACC GGG GCT CTA ATA ACT CCT TGT AGC CCT GAG GAG GAG AAG 480
Ser Trp Thr Gly Ala Leu Ile Thr Pro Cys Ser Pro Glu Glu Glu Lys
145 150 155 160

TTG CCA ATT AAC CCC TTG AGT AAC TCG CTG TTG CGA TAC CAC AAC AAG 528
Leu Pro Ile Asn Pro Leu Ser Asn Ser Leu Leu Arg Tyr His Asn Lys
165 170 175

GTG TAT TGC ACT ACA ACA AGA AGC GCG GAA TTC 561
Val Tyr Cys Thr Thr Thr Arg Ser Ala Glu Phe
180 185

SEQ ID NO: 4
SEQUENCE LENGTH: 403
SEQUENCE TYPE: nucleic acid
STRANDEDNESS: double
TOPOLOGY: linear
MOLECULE TYPE: cDNA to genomic RNA
ORIGINAL SOURCE: non-A, non-B hepatitis virus
SEQUENCE DESCRIPTION:

```
GAA TTC CTC GTC ACT AGT ACC TGG GTG CTA GTA GGC GGA GTC CTT GCA     48
Glu Phe Leu Val Thr Ser Thr Trp Val Leu Val Gly Gly Val Leu Ala
  1               5                  10                  15

GCT TTG GCC GCG TAT TGC CTA ACA ACA GGC AGC GTG GTC ATT GTA GGT     96
Ala Leu Ala Ala Tyr Cys Leu Thr Thr Gly Ser Val Val Ile Val Gly
             20                  25                  30

AGG ATC ATC TTG TCC GGG AGG CCG GCT GTC ATT CCC GAT AGG GAG GTT    144
Arg Ile Ile Leu Ser Gly Arg Pro Ala Val Ile Pro Asp Arg Glu Val
         35                  40                  45

CTC TAC CGA GAG TTC GAT GAA ATG GAA GAG TGC GCC TCA CAC CTC CCT    192
Leu Tyr Arg Glu Phe Asp Glu Met Glu Glu Cys Ala Ser His Leu Pro
     50                  55                  60

TAC ATC GAA CAA GGG ATG CAG CTC GCC GAG CAA TTC AAA CAG AAG GCG    240
Tyr Ile Glu Gln Gly Met Gln Leu Ala Glu Gln Phe Lys Gln Lys Ala
 65                  70                  75                  80
```

```
CTC GGG TTG CTG CAA ACA GCC GCC AAG CAA GCC GAG GTT GCT GCT CCC   288
Leu Gly Leu Leu Gln Thr Ala Ala Lys Gln Ala Glu Val Ala Ala Pro
                 85                  90                  95

GTG GTG GAA TCC AAG TGG CGA GCC CTC GAA GCC TTC TGG GCG AAG CAC   336
Val Val Glu Ser Lys Trp Arg Ala Leu Glu Ala Phe Trp Ala Lys His
            100                 105                 110

ATG TGG AAT TTC ATC AGC GGG ATA CAG TAC TTA GCA GGC TTG TCC ACT   384
Met Trp Asn Phe Ile Ser Gly Ile Gln Tyr Leu Ala Gly Leu Ser Thr
        115                 120                 125

CTG CCT GGA AAC GGA ATT C                                          403
Leu Pro Gly Asn Gly Ile
    130
```

SEQ ID NO: 5
SEQUENCE LENGTH: 132
SEQUENCE TYPE: nucleic acid
STRANDEDNESS: double
TOPOLOGY: linear
MOLECULE TYPE: cDNA to genomic RNA
ORIGINAL SOURCE: non-A, non-B hepatitis virus
SEQUENCE DESCRIPTION:

```
GAA TTC CGT GGG CCC GAG GAG GAG AAG TTG CCG ATC AAC CCT CTG AGT    48
Glu Phe Arg Gly Pro Glu Glu Glu Lys Leu Pro Ile Asn Pro Leu Ser
 1               5                  10                  15

AAC TCG CTC ATG CGG TTT CAT AAC AAG GTG TAC TCC ACA ACT TCG AGG    96
Asn Ser Leu Met Arg Phe His Asn Lys Val Tyr Ser Thr Thr Ser Arg
            20                  25                  30

AGT GCC ACT CTG AGG GCA AAG AAG GTG ACG GAA TTC                   132
Ser Ala Thr Leu Arg Ala Lys Lys Val Thr Glu Phe
        35                  40
```

SEQ ID NO: 6
SEQUENCE LENGTH: 964
SEQUENCE TYPE: nucleic acid
STRANDEDNESS: double
TOPOLOGY: linear
MOLECULE TYPE: cDNA to genomic RNA
ORIGINAL SOURCE: non-A, non-B hepatitis virus
SEQUENCE DESCRIPTION:

```
GAA TTC CGA AAA ACC AAA CGT AAC ACC AAC CGC CGC CCA CAG GAC GTC    48
Glu Phe Arg Lys Thr Lys Arg Asn Thr Asn Arg Arg Pro Gln Asp Val
  1               5                  10                  15

AAG TTC CCG GGC GGT GGT CAG ATC GTT GGT GGA GTT TAC CTG TTG CCG    96
Lys Phe Pro Gly Gly Gly Gln Ile Val Gly Gly Val Tyr Leu Leu Pro
             20                  25                  30

CGC AGG GGC CCC AGG TTG GGT GTG CGC GCG ACT AGG AAG ACT TCC GAG   144
Arg Arg Gly Pro Arg Leu Gly Val Arg Ala Thr Arg Lys Thr Ser Glu
         35                  40                  45

CGG TCG CAA CCT CGT GGA AGG CGA CAA CAT ATC CCC AAG GCT CGC CGG   192
Arg Ser Gln Pro Arg Gly Arg Arg Gln His Ile Pro Lys Ala Arg Arg
     50                  55                  60

CCC GAG GGC AGG GCC TGG GCT CAG CCC GGG TAC CCT TGG CCC CTC TAT   240
Pro Glu Gly Arg Ala Trp Ala Gln Pro Gly Tyr Pro Trp Pro Leu Tyr
 65                  70                  75                  80
```

```
GGC AAT GAG GGC TTG GGG TGG GCA GGA TGG CTC CTG TCA CCC CGC GGC   288
Gly Asn Glu Gly Leu Gly Trp Ala Gly Trp Leu Leu Ser Pro Arg Gly
                 85                  90                  95

TCC CGG CCT AGT TGG GGC CCT ACA GAC CCC CGG CGT AGG TCG CGT AAT   336
Ser Arg Pro Ser Trp Gly Pro Thr Asp Pro Arg Arg Arg Ser Arg Asn
            100                 105                 110

TTG GGT AAG GTC ATC GAC ACC CTC ACA TGC GGC TTC GCC GAC CTC ATG   384
Leu Gly Lys Val Ile Asp Thr Leu Thr Cys Gly Phe Ala Asp Leu Met
        115                 120                 125

GGG TAC ATC CCG CTC GTC GGC GCC CCC CTG GGG GGC GCT GCC AGG GCC   432
Gly Tyr Ile Pro Leu Val Gly Ala Pro Leu Gly Gly Ala Ala Arg Ala
    130                 135                 140

CTG GCA CAT GGT GTC CGG GTT CTG GAG GAC GGC GTG AAC TAT GCA ACA   480
Leu Ala His Gly Val Arg Val Leu Glu Asp Gly Val Asn Tyr Ala Thr
145                 150                 155                 160

GGG AAT CTG CCC GGT TGC TCT TTC TCT ATC TTC CTC CTG GCT CTG CTG   528
Gly Asn Leu Pro Gly Cys Ser Phe Ser Ile Phe Leu Leu Ala Leu Leu
                165                 170                 175

TCC TGT TTG ACC ATC CCA GCT TCC GCT TAT GAA GTG CGC AAC GTG TCC   576
Ser Cys Leu Thr Ile Pro Ala Ser Ala Tyr Glu Val Arg Asn Val Ser
            180                 185                 190
```

GGG GCG TAC CAC GTC ACG AAC GAC TGC TCC AAC TCA AGC ATT GTG TAT 624
Gly Ala Tyr His Val Thr Asn Asp Cys Ser Asn Ser Ser Ile Val Tyr
195 200 205

GAG GCA GCG GAC GTG ATC ATG CAC ACC CCC GGG TGC GTG CCC TGC GTT 672
Glu Ala Ala Asp Val Ile Met His Thr Pro Gly Cys Val Pro Cys Val
210 215 220

CGG GAG AGC AAC TCC TCC CGT TGC TGG GTA GCG CTC ACC CCC ACG CTC 720
Arg Glu Ser Asn Ser Ser Arg Cys Trp Val Ala Leu Thr Pro Thr Leu
225 230 235 240

GCG GCT AGA AAT ACC AGC ATC CCC ACT ACG ACA ATA CGA CGC CAT GTC 768
Ala Ala Arg Asn Thr Ser Ile Pro Thr Thr Thr Ile Arg Arg His Val
245 250 255

GAC TTG CTC GTT GGG GCG GCT GCT TTC TGC TCC GCT ATG TAT GTA GGG 816
Asp Leu Leu Val Gly Ala Ala Ala Phe Cys Ser Ala Met Tyr Val Gly
260 265 270

GAT CTC TGC GGA TCT GTT TTC CTT GTC TCT CAG CTG TTC ACC TTC TCG 864
Asp Leu Cys Gly Ser Val Phe Leu Val Ser Gln Leu Phe Thr Phe Ser
275 280 285

CCT CGC CGG TAC CAG ACA GTA CAG GAC TGC AAT TGT TCA ATC TAT CCC 912
Pro Arg Arg Tyr Gln Thr Val Gln Asp Cys Asn Cys Ser Ile Tyr Pro
290 295 300

GGC CAC CTA TCA GGT CAC CGC ATG GCT TGG GAT ATG ATG ATG AAC GGA 960
Gly His Leu Ser Gly His Arg Met Ala Trp Asp Met Met Met Asn Gly
305 310 315 320

ATT C 964
Ile

SEQ ID NO: 7
SEQUENCE LENGTH: 1009
SEQUENCE TYPE: nucleic acid
STRANDEDNESS: double
TOPOLOGY: linear
MOLECULE TYPE: cDNA to genomic RNA
ORIGINAL SOURCE: non-A, non-B hepatitis virus
SEQUENCE DESCRIPTION:

```
GAA TTC TTC TCT TGG GTG GAC GGG GTG CAA ATA CAC CGA TTC GTC CCC    48
Glu Phe Phe Ser Trp Val Asp Gly Val Gln Ile His Arg Phe Val Pro
  1               5                  10                  15

ACT CCG GGC CCC TTC TTT CGG GAT GAG GTA ACG TTC ACC GTA GGC CTC    96
Thr Pro Gly Pro Phe Phe Arg Asp Glu Val Thr Phe Thr Val Gly Leu
            20                  25                  30

AAT TTC TTT GTG GTC GGC TCC CAG CTC CCT TGT GAC CCT GAG CCG GAC   144
Asn Phe Phe Val Val Gly Ser Gln Leu Pro Cys Asp Pro Glu Pro Asp
        35                  40                  45

ACC GAG GCG CTA GCC TCC ATG CTG ACA GAC CCG TCC CAC ATT ACT GCG   192
Thr Glu Ala Leu Ala Ser Met Leu Thr Asp Pro Ser His Ile Thr Ala
    50                  55                  60

GAG ACG GCA GCC AGA CGA TTG GCC AGG GGA TCT CCC CCT TCA CAG GCC   240
Glu Thr Ala Ala Arg Arg Leu Ala Arg Gly Ser Pro Pro Ser Gln Ala
 65                  70                  75                  80
```

AGC TCT TCA GCG AGC CAG CTC TCC GCC CCG TCC TTG AAG GCT ACG TGC 288
Ser Ser Ser Ala Ser Gln Leu Ser Ala Pro Ser Leu Lys Ala Thr Cys
85 90 95

ACC ACC CAT AAG ATG GCA TAT GAC TGT GAC ATG GTG GAT GCT AAC CTT
Thr Thr His Lys Met Ala Tyr Asp Cys Asp Met Val Asp Ala Asn Leu
100 105 110

TTC ATG GGA GGT GAT GTG ACC CGG ATT GAG TCC GAC TCC AAG GTA ATC 384
Phe Met Gly Gly Asp Val Thr Arg Ile Glu Ser Asp Ser Lys Val Ile
115 120 125

GTT CTC GAC TCC CTC GAC TCC ATG ACT GAG GTA GAG GAT GAA CGT GAG 432
Val Leu Asp Ser Leu Asp Ser Met Thr Glu Val Glu Asp Glu Arg Glu
130 135 140

CCT TCT GTA CCA TCA GAG TAC TTG ATC AGG AGG AGG AAG TTC CCA CCG 480
Pro Ser Val Pro Ser Glu Tyr Leu Ile Arg Arg Arg Lys Phe Pro Pro
145 150 155 160

GCA CTA CCT CCC TGG GCC CGT CCG GAC TAT AAC CCT CCT ACG ATC GAG 528
Ala Leu Pro Pro Trp Ala Arg Pro Asp Tyr Asn Pro Pro Thr Ile Glu
165 170 175

ATA TGG AAG AGG CCG GGC TAT GAA CCA CCT ACT GTC CTA GGC TGT GCC 576
Ile Trp Lys Arg Pro Gly Tyr Glu Pro Pro Thr Val Leu Gly Cys Ala
180 185 190

CTC CCC CCC ACG CCT CAA GCG CCA GTG CCC CCA CCC AGG AAG CGC CGC 624
Leu Pro Pro Thr Pro Gln Ala Pro Val Pro Pro Pro Arg Lys Arg Arg
200 205 210

GCC AAA GTC CTG ACT CAG GAC AAC GTG GAG GGG GTC CTT AGG GAG ATG 672
Ala Lys Val Leu Thr Gln Asp Asn Val Glu Gly Val Leu Arg Glu Met
215 220 225

GCG GAC AAG GTG CTC AGT CCT TCC CAA GAC CAC AAT GAC TCC GGT CAC 720
Ala Asp Lys Val Leu Ser Pro Ser Gln Asp His Asn Asp Ser Gly His
230 235 240 245

TCC ACC GGA GCG GAC ACC GGA GGA GAC AGC TTC CAG CAG CTC TTC GAC 768
Ser Thr Gly Ala Asp Thr Gly Gly Asp Ser Phe Gln Gln Leu Phe Asp
250 255 260

GAG ACT GCC GCT TCA GAA GCG GGA TCA CTG TCC TCC ATG CCT CCC CTT 816
Glu Thr Ala Ala Ser Glu Ala Gly Ser Leu Ser Ser Met Pro Pro Leu
265 270 275

GAG GGG GAG CCG GGG GAC CCT GAC CTG GAG TTT GAA CCA GCG GGA TCC 864
Glu Gly Glu Pro Gly Asp Pro Asp Leu Glu Phe Glu Pro Ala Gly Ser
280 285 290

GGT CCC CCT TCT GAG GGG GAG TGC GAG GTC GTT GAT TCG GAC TCT AAG 912
Gly Pro Pro Ser Glu Gly Glu Cys Glu Val Val Asp Ser Asp Ser Lys
295 300 305

TCG TGG TCC ACA GTC TCG GAT CAG GAG GAT TCT GTT ATC TGC TGC TCC 960

Ser Trp Ser Thr Val Ser Asp Gln Glu Asp Ser Val Ile Cys Cys Ser

310 315 320 325

ATG TCA TAC TCC TGG ACA GGG GCC CTC ATA ACA CCA TGT GGG GGA ATT 1008

Met Ser Tyr Ser Trp Thr Gly Ala Leu Ile Thr Pro Cys Gly Gly Ile

330 335 340

C 1009

SEQ ID NO: 8
SEQUENCE LENGTH: 608
SEQUENCE TYPE: nucleic acid
STRANDEDNESS: double
TOPOLOGY: linear
MOLECULE TYPE: cDNA to genomic RNA
ORIGINAL SOURCE: non-A, non-B hepatitis virus
SEQUENCE DESCRIPTION:

```
GAA TTC CCG CGG GGG TCA CCC CCG TCT GAG GCA AGT TCC TCA GCG AGC    48
Glu Phe Pro Arg Gly Ser Pro Pro Ser Glu Ala Ser Ser Ser Ala Ser
  1               5                  10                  15

CAA CTA TCG GCA CCA TCG CTG CGA GCC ACC TGT ACC ACC CAC GGC AAG    96
Gln Leu Ser Ala Pro Ser Leu Arg Ala Thr Cys Thr Thr His Gly Lys
                 20                  25                  30

ACC TAC GAT GTG GAC ATG GTG GAT GCT AAC CTG TTT ATG GGA GGC GAT   144
Thr Tyr Asp Val Asp Met Val Asp Ala Asn Leu Phe Met Gly Gly Asp
             35                  40                  45

GTG ACT CGG ATA GAA TCT GAA TCC AAA GTG GTC GTT CTG GAC TCC CTC   192
Val Thr Arg Ile Glu Ser Glu Ser Lys Val Val Val Leu Asp Ser Leu
         50                  55                  60

GAC CCA ATG GCC GAA GAA ATG AGC GAC CTC GAG CCT TCT ATA CCA TCG   240
Asp Pro Met Ala Glu Glu Met Ser Asp Leu Glu Pro Ser Ile Pro Ser
 65                  70                  75                  80
```

GAG TAT ATG CTC CCC AAA ACC AGG TTC CCA CCA GCC TTA CCG GCC TGG 288

Glu Tyr Met Leu Pro Lys Thr Arg Phe Pro Pro Ala Leu Pro Ala Trp

85 90 95

GCA CGG CCT GAC TAC AAC CCA CCG TTT GTG GAA CCA TGG AGG AGA CCA 336

Ala Arg Pro Asp Tyr Asn Pro Pro Phe Val Glu Pro Trp Arg Arg Pro

100 105 110

GAC TAC CAA CCG CCC ACT GTT GCG GGC TGT GCT CTC CCC CCC CCC AAG 384

Asp Tyr Gln Pro Pro Thr Val Ala Gly Cys Ala Leu Pro Pro Pro Lys

115 120 125

AAG ACC CCG ACG CCC CCC CCA AGG AGA CGC CGG ACA GTG GGT CTG AGT 432

Lys Thr Pro Thr Pro Pro Pro Arg Arg Arg Arg Thr Val Gly Leu Ser

130 135 140

GAG AGC ACC ATA GGA GAT GTC CTC CAA CAG ATG GCC ATT AAG ACC TTT 480

Glu Ser Thr Ile Gly Asp Val Leu Gln Gln Met Ala Ile Lys Thr Phe

145 150 155 160

GGC CAG CCC CCC CCA AGC GGA GAT TCA GGC CTC TCT ACG GGG GCG GAC 528

Gly Gln Pro Pro Pro Ser Gly Asp Ser Gly Leu Ser Thr Gly Ala Asp

165 170 175

GCC GCC GAC TCT GGT AGT CGG ACG CCC CCC GAG GAG TTA GCT CTC TCG 576

Ala Ala Asp Ser Gly Ser Arg Thr Pro Pro Glu Glu Leu Ala Leu Ser

180 185 190

GAG ACA GGT TCT ACC TCC TCC ATG CGG AAT TC 608

Glu Thr Gly Ser Thr Ser Ser Met Arg Asn

195 200

SEQ ID NO: 9
SEQUENCE LENGTH: 897
SEQUENCE TYPE: nucleic acid
STRANDEDNESS: double
TOPOLOGY: linear
MOLECULE TYPE: cDNA to genomic RNA
ORIGINAL SOURCE: non-A, non-B hepatitis virus
SEQUENCE DESCRIPTION:

GAA TTC CGG CGG GGG GAC GGC ATC ATG CAA ACC ACC TGC CCA TGT GGA 48

Glu Phe Arg Arg Gly Asp Gly Ile Met Gln Thr Thr Cys Pro Cys Gly

1 5 10 15

GCA CAG ATC ACC GGA CAT GTC AAA AAC GGT TCC ATG AGG ATT GTT GGG 96

Ala Gln Ile Thr Gly His Val Lys Asn Gly Ser Met Arg Ile Val Gly

20 25 30

CCT AAA ACC TGT AGC AAC ACG TGG CAT GGA ACA TTC CCC ATC AAT GCA 144

Pro Lys Thr Cys Ser Asn Thr Trp His Gly Thr Phe Pro Ile Asn Ala

35 40 45

TAC ACC ACG GGC CCC TGC ACA CCC TCC CCA GCA CCA AAC TAT TCT AGG 192

Tyr Thr Thr Gly Pro Cys Thr Pro Ser Pro Ala Pro Asn Tyr Ser Arg

50 55 60

GCG CTG TGG CGG GTG GCT GCT GAG GAG TAC GTG GAG GTT ACG CGG GTG 240

Ala Leu Trp Arg Val Ala Ala Glu Glu Tyr Val Glu Val Thr Arg Val

65 70 75 80

GGG GAC TTC CAC TAC GTG ACG GGC ATG ACC ACT GAC AAC ATA AAG TGC 288
Gly Asp Phe His Tyr Val Thr Gly Met Thr Thr Asp Asn Ile Lys Cys
85 90 95

CCA TGC CAG GTT CCG GCC CCC GAG TTC TTC ACG GAG GTG GAT GGA GTG 336
Pro Cys Gln Val Pro Ala Pro Glu Phe Phe Thr Glu Val Asp Gly Val
100 105 110

AGG TTG CAC AGG TAC GCT CCG GCA TGC AAA CCT CTC CTA CGG GAT GAG 384
Arg Leu His Arg Tyr Ala Pro Ala Cys Lys Pro Leu Leu Arg Asp Glu
115 120 125

GTC ACA TTC CAG GTC GGG CTC AAC CAG TAC CTG GTA GGG TCA CAG CTC 432
Val Thr Phe Gln Val Gly Leu Asn Gln Tyr Leu Val Gly Ser Gln Leu
130 135 140

CCA TGT GAG CCC GAA CCG GAC GTA GCT GTG CTT ACT TCC ATG CTC ACC 480
Pro Cys Glu Pro Glu Pro Asp Val Ala Val Leu Thr Ser Met Leu Thr
145 150 155 160

GAT CCC TCC CAC ATT ACA GCA GAG ACG GCC AAG CGT AGG CTG GCC AGG 528
Asp Pro Ser His Ile Thr Ala Glu Thr Ala Lys Arg Arg Leu Ala Arg
165 170 175

GGG TCT CCC CCC TCC TTG GCC AGC TCT TCA GCT AGC CAG TTG TCT GCG 576
Gly Ser Pro Pro Ser Leu Ala Ser Ser Ser Ala Ser Gln Leu Ser Ala
180 185 190

CCT TCC TTG AAG GCG ACA TGT ACT ACC CAT CAT GAC TCC CCG GAA GCT 624
Pro Ser Leu Lys Ala Thr Cys Thr Thr His His Asp Ser Pro Glu Ala
195 200 205

GAC CTC ATC GAG GCC AAC CTC CTG TGG CGG CAG GAG ATG GGC GGG AAC 672
Asp Leu Ile Glu Ala Asn Leu Leu Trp Arg Gln Glu Met Gly Gly Asn
210 215 220

ATC ACC CGC GTG GAG TCA GAA AAT AAG GTA GTA ATT CTG GAC TCT TTC 720
Ile Thr Arg Val Glu Ser Glu Asn Lys Val Val Ile Leu Asp Ser Phe
225 230 235 240

GAT CCG CTC CGG GCG GAG GAG GAT GAG AGG GAA ATG TCC ATT CCG GCG 768
Asp Pro Leu Arg Ala Glu Glu Asp Glu Arg Glu Met Ser Ile Pro Ala
245 250 255

GAG ATC CTG CGG AAA CCC AGG AGG TTC CCC CCA GCA TTG CCC ATA TGG 816
Glu Ile Leu Arg Lys Pro Arg Arg Phe Pro Pro Ala Leu Pro Ile Trp
260 265 270

GCA CGT GCG GAT TAC AAC CCT CCA CTG ATA GAA CCC TGG AAG GAC CCG 864
Ala Arg Ala Asp Tyr Asn Pro Pro Leu Ile Glu Pro Trp Lys Asp Pro
275 280 285

GAC TAC GTC CCT CCG GTG GTG CAC GGG GAA TTC 897
Asp Tyr Val Pro Pro Val Val His Gly Glu Phe
290 295

SEQ ID NO: 10
SEQUENCE LENGTH: 218
SEQUENCE TYPE: nucleic acid
STRANDEDNESS: double
TOPOLOGY: linear
MOLECULE TYPE: cDNA to genomic RNA
ORIGINAL SOURCE: non-A, non-B hepatitis virus
SEQUENCE DESCRIPTION:

```
GAA TTC TTC TCT TGG GTG GAC GGG GTG CAA ATA CAC CGA TTC GCC CCC      48
Glu Phe Phe Ser Trp Val Asp Gly Val Gln Ile His Arg Phe Ala Pro
  1               5                  10                  15
ACT CCA GGT CCC TTC TTT CGG GAT GAG GTA ACG TTC ACC GTG GGC CTC      96
Thr Pro Gly Pro Phe Phe Arg Asp Glu Val Thr Phe Thr Val Gly Leu
             20                  25                  30
AAT TCC TTT GTG GTC GGC TCT CAG CTC CCC TGC GAC CCT GAG CCG GAC     144
Asn Ser Phe Val Val Gly Ser Gln Leu Pro Cys Asp Pro Glu Pro Asp
         35                  40                  45
ACC GAG GTA TTA GCC TCC ATG TTG ACA GAC CCG TCC CAC ATC ACC GCG     192
Thr Glu Val Leu Ala Ser Met Leu Thr Asp Pro Ser His Ile Thr Ala
     50                  55                  60
GAG GCG GCA GCC AGG CGG TTG GCC AG                                  218
Glu Ala Ala Ala Arg Arg Leu Ala
 65                  70
```

SEQ ID NO: 11
SEQUENCE LENGTH: 66
SEQUENCE TYPE: nucleic acid
STRANDEDNESS: double
TOPOLOGY: linear
MOLECULE TYPE: cDNA to genomic RNA
ORIGINAL SOURCE: non-A, non-B hepatitis virus
SEQUENCE DESCRIPTION:

```
                                                                GC      2
CGC CCA CAG GAC GTC AAG TTC CCG GGC GGT GGT CAG ATC GTT GGT GGA        50
Arg Pro Gln Asp Val Lys Phe Pro Gly Gly Gly Gln Ile Val Gly Gly
  1               5                  10                  15
GTT TAC CTG TTG CCG C                                                  66
Val Tyr Leu Leu Pro
                 20
```

SEQ ID NO: 12
SEQUENCE LENGTH: 181
SEQUENCE TYPE: nucleic acid
STRANDEDNESS: double
TOPOLOGY: linear
MOLECULE TYPE: cDNA to genomic RNA
ORIGINAL SOURCE: non-A, non-B hepatitis virus
SEQUENCE DESCRIPTION:

```
                                                      CGTGCACC     8

ATG AGC ACA AAT CCT AAA CCT CAA AGA AAA ACC AAA AGA AAT ACT AAC   56
Met Ser Thr Asn Pro Lys Pro Gln Arg Lys Thr Lys Arg Asn Thr Asn
 1               5                  10                  15
CGT CGC CCA CAA GAC GTC AAG TTT CCG GGC GGC GGC CAG ATC GTT GGC  104
Arg Arg Pro Gln Asp Val Lys Phe Pro Gly Gly Gly Gln Ile Val Gly
            20                  25                  30
GGA GTA TAC TTG TTG CCG CGC AGG GGC CCT AGG TTG GGT GTG CGC ACG  152
Gly Val Tyr Leu Leu Pro Arg Arg Gly Pro Arg Leu Gly Val Arg Thr
        35                  40                  45
ACA AGG AAG ACT TCG GAG CGA TCC CAG CC                           181
Thr Arg Lys Thr Ser Glu Arg Ser Gln
    50                  55
```

SEQ ID NO: 13
SEQUENCE LENGTH: 561
SEQUENCE TYPE: nucleic acid
STRANDEDNESS: single
TOPOLOGY: linear
MOLECULE TYPE: Genomic RNA
ORIGINAL SOURCE: non-A, non-B hepatitis virus
SEQUENCE DESCRIPTION:

```
GAAUUCCAUU ACCUACCGCC UACUGUUGCA GGCUGUGCUC UCCCCCCCCC CAAGAAGACC  60
CCGACGCCCC CCCCGAGGAG GCGCCGGACA GUGGCCUUGG ACGGGAGCAC CAUUGGAGAC 120
GUCCUCCAGC AGUUGGCCGU CAAGACCUUC GGCCAGCCCC CCCCGAGCGG CGACUCGGGC 180
CCCUCCACGG GGGCGGACGU CGUCGGCUCU GGUGGUCGGA CGCCCCCUGA UGAAUUGGCU 240
CUCUCGGAGA CAGGUUCUGU CUCUUCCAUG CCCCCCCUCG AGGGGGAGCC UGGGGAUCCG 300
GACCUAGAGC CUGAGCGGGU AGAGCUUCAG CCCCCCCCCC AGGGGGGGGA GGAAGCUCCC 360
GGCUCGGACU CGGGGUCCUG GUCUACUUGC UCCGAGGAGA GUGACUCCGU CGUGUGCUGC 420
UCCAUGUCUU ACUCCUGGAC CGGGGCUCUA AUAACUCCUU GUAGCCCUGA GGAGGAGAAG 480
UUGCCAAUUA ACCCCUUGAG UAACUCGCUG UUGCGAUACC ACAACAAGGU GUAUUGCACU 540
ACAACAAGAA GCGCGGAAUU C                                            561
```

SEQ ID NO: 14
SEQUENCE LENGTH: 403
SEQUENCE TYPE: nucleic acid
STRANDEDNESS: single
TOPOLOGY: linear
MOLECULE TYPE: Genomic RNA
ORIGINAL SOURCE: non-A, non-B hepatitis virus
SEQUENCE DESCRIPTION:

GAAUUCCUCG UCACUAGUAC CUGGGUGCUA GUAGGCGGAG UCCUUGCAGC UUUGGCCGCG 60
UAUUGCCUAA CAACAGGCAG CGUGGUCAUU GUAGGUAGGA UCAUCUUGUC CGGGAGGCCG 120
GCUGUCAUUC CCGAUAGGGA GGUUCUCUAC CGAGAGUUCG AUGAAAUGGA AGAGUGCGCC 180
UCACACCUCC CUUACAUCGA ACAAGGGAUG CAGCUCGCCG AGCAAUUCAA ACAGAAGGCG 240
CUCGGGUUGC UGCAAACAGC CGCCAAGCAA GCGGAGGUUG CUGCUCCCGU GGUGGAAUCC 300
AAGUGGCGAG CCCUCGAAGC CUUCUGGGCG AAGCACAUGU GGAAUUUCAU CAGCGGGAUA 360
CAGUACUUAG CAGGCUUGUC CACUCUGCCU GGAAACGGAA UUC 403

SEQ ID NO: 15
SEQUENCE LENGTH: 132
SEQUENCE TYPE: nucleic acid
STRANDEDNESS: single
TOPOLOGY: linear
MOLECULE TYPE: Genomic RNA
ORIGINAL SOURCE: non-A, non-B hepatitis virus
SEQUENCE DESCRIPTION:

GAAUUCCGUG GGCCCGAGGA GGAGAAGUUG CCGAUCAACC CUCUGAGUAA CUCGCUCAUG 60
CGGUUUCAUA ACAAGGUGUA CUCCACAACU UCGAGGAGUG CCACUCUGAG GGCAAAGAAG 120
GUGACGGAAU UC 132

SEQ ID NO: 16
SEQUENCE LENGTH: 964
SEQUENCE TYPE: nucleic acid
STRANDEDNESS: single
TOPOLOGY: linear
MOLECULE TYPE: Genomic RNA
ORIGINAL SOURCE: non-A, non-B hepatitis virus
SEQUENCE DESCRIPTION:

```
GAAUUCCGAA AAACCAAACG UAACACCAAC CGCCGCCCAC AGGACGUCAA GUUCCCGGGC  60
GGUGGUCAGA UCGUUGGUGG AGUUUACCUG UUGCCGCGCA GGGGCCCCAG GUUGGGUGUG 120
CGCGCGACUA GGAAGACUUC CGAGCGGUCG CAACCUCGUG GAAGGCGACA ACAUAUCCCC 180
AAGGCUCGCC GGCCCGAGGG CAGGGCCUGG GCUCAGCCCG GGUACCCUUG GCCCCUCUAU 240
GGCAAUGAGG GCUUGGGGUG GGCAGGAUGG CUCCUGUCAC CCCGCGGCUC CCGGCCUAGU 300
UGGGGCCCUA CAGACCCCCG GCGUAGGUCG CGUAAUUUGG GUAAGGUCAU CGACACCCUC 360
ACAUGCGGCU UCGCCGACCU CAUGGGGUAC AUCCCGCUCG UCGGCGCCCC CCUGGGGGGC 420
GCUGCCAGGG CCCUGGCACA UGGUGUCCGG GUUCUGGAGG ACGGCGUGAA CUAUGCAACA 480
GGGAAUCUGC CCGGUUGCUC UUUCUCUAUC UUCCUCCUGG CUCUGCUGUC CUGUUUGACC 540
AUCCCAGCUU CCGCUUAUGA AGUGCGCAAC GUGUCCGGGG CGUACCACGU CACGAACGAC 600
UGCUCCAACU CAAGCAUUGU GUAUGAGGCA GCGGACGUGA UCAUGCACAC CCCCGGGUGC 660
GUGCCCUGCG UUCGGGAGAG CAACUCCUCC CGUUGCUGGG UAGCGCUCAC CCCCACGCUC 720
GCGGCUAGAA AUACCAGCAU CCCCACUACG ACAAUACGAC GCCAUGUCGA CUUGCUCGUU 780
GGGGCGGCUG CUUUCUGCUC CGCUAUGUAU GUAGGGGAUC UCUGCGGAUC UGUUUUCCUU 840
GUCUCUCAGC UGUUCACCUU CUCGCCUCGC CGGUACCAGA CAGUACAGGA CUGCAAUUGU 900
UCAAUCUAUC CCGGCCACCU AUCAGGUCAC CGCAUGGCUU GGGAUAUGAU GAUGAACGGA 960
AUUC                                                             964
```

SEQ ID NO: 17
SEQUENCE LENGTH: 1009
SEQUENCE TYPE: nucleic acid
STRANDEDNESS: single
TOPOLOGY: linear
MOLECULE TYPE: Genomic RNA
ORIGINAL SOURCE: non-A, non-B hepatitis virus
SEQUENCE DESCRIPTION:

```
GAAUUCUUCU CUUGGGUGGA CGGGGUGCAA AUACACCGAU UCGUCCCCAC UCCGGGCCCC   60
UUCUUUCGGG AUGAGGUAAC GUUCACCGUA GGCCUCAAUU UCUUGUGGU CGGCUCCCAG  120
CUCCCUUGUG ACCCUGAGCC GGACACCGAG GCGCUAGCCU CCAUGCUGAC AGACCCGUCC  180
CACAUUACUG CGGAGACGGC AGCCAGACGA UUGGCCAGGG GAUCUCCCCC UUCACAGGCC  240
AGCUCUUCAG CGAGCCAGCU CUCCGCCCCG UCCUUGAAGG CUACCUGCAC CACCCAUAAG  300
AUGGCAUAUG ACUGUGACAU GGUGGAUGCU AACCUUUUCA UGGGAGGUGA UGUGACCCGG  360
AUUGAGUCCG ACUCCAAGGU AAUCGUUCUC GACUCCCUCG ACUCCAUGAC UGAGGUAGAG  420
GAUGAACGUG AGCCUUCUGU ACCAUCAGAG UACUUGAUCA GGAGGAGGAA GUUCCCACCG  480
GCACUACCUC CCUGGGCCCG UCCGGACUAU AACCCUCCUA CGAUCGAGAU AUGGAAGAGG  540
CCGGGCUAUG AACCACCUAC UGUCCUAGGC UGUGCCCUCC CCCCCACGCC UCAAGCGCCA  600
GUGCCCCCAC CCAGGAAGCG CCGCGCCAAA GUCCUGACUC AGGACAACGU GGAGGGGGUC  660
CUUAGGGAGA UGGCGGACAA GGUGCUCAGU CCUUCCCAAG ACCACAAUGA CUCCGGUCAC  720
UCCACCGGAG CGGACACCGG AGGAGACAGC UUCCAGCAGC UCUUCGACGA GACUGCCGCU  780
UCAGAAGCGG GAUCACUGUC CUCCAUGCCU CCCCUUGAGG GGGAGCCGGG GGACCCUGAC  840
CUGGAGUUUG AACCAGCGGG AUCCGGUCCC CCUUCUGAGG GGGAGUGCGA GGUCGUUGAU  900
UCGGACUCUA AGUCGUGGUC CACAGUCUCG GAUCAGGAGG AUUCUGUUAU CUGCUGCUCC  960
AUGUCAUACU CCUGGACAGG GGCCCUCAUA ACACCAUGUG GGGGAAUUC            1009
```

SEQ ID NO: 18
SEQUENCE LENGTH: 608
SEQUENCE TYPE: nucleic acid
STRANDEDNESS: single
TOPOLOGY: linear
MOLECULE TYPE: Genomic RNA
ORIGINAL SOURCE: non-A, non-B hepatitis virus
SEQUENCE DESCRIPTION:

```
GAAUUCCCGC GGGGGUCACC CCCGUCUGAG GCAAGUUCCU CAGCGAGCCA ACUAUCGGCA  60
CCAUCGCUGC GAGCCACCUG UACCACCCAC GGCAAGACCU ACGAUGUGGA CAUGGUGGAU 120
GCUAACCUGU UUAUGGGAGG CGAUGUGACU CGGAUAGAAU CUGAAUCCAA AGUGGUCGUU 180
CUGGACUCCC UCGACCCAAU GGCCGAAGAA AUGAGCGACC UCGAGCCUUC UAUACCAUCG 240
GAGUAUAUGC UCCCCAAAAC CAGGUUCCCA CCAGCCUUAC CGGCCUGGGC ACGGCCUGAC 300
UACAACCCAC CGUUUGUGGA ACCAUGGAGG AGACCAGACU ACCAACCGCC CACUGUUGCG 360
GGCUGUGCUC UCCCCCCCCC CAAGAAGACC CCGACGCCCC CCCCAAGGAG ACGCCGGACA 420
GUGGGUCUGA GUGAGAGCAC CAUAGGAGAU GUCCUCCAAC AGAUGGCCAU UAAGACCUUU 480
GGCCAGCCCC CCCCAAGCGG AGAUUCAGGC CUCUCUACGG GGGCGGACGC CGCCGACUCU 540
GGUAGUCGGA CGCCCCCCGA GGAGUUAGCU CUCUCGGAGA CAGGUUCUAC CUCCUCCAUG 600
CGGAAUUC                                                          608
```

SEQ ID NO: 19
SEQUENCE LENGTH: 897
SEQUENCE TYPE: nucleic acid
STRANDEDNESS: single
TOPOLOGY: linear
MOLECULE TYPE: Genomic RNA
ORIGINAL SOURCE: non-A, non-B hepatitis virus
SEQUENCE DESCRIPTION:

```
GAAUUCCGGC GGGGGGACGG CAUCAUGCAA ACCACCUGCC CAUGUGGAGC ACAGAUCACC  60
GGACAUGUCA AAAACGGUUC CAUGAGGAUU GUUGGGCCUA AAACCUGUAG CAACACGUGG 120
CAUGGAACAU UCCCCAUCAA UGCAUACACC ACGGGCCCCU GCACACCCUC CCCAGCACCA 180
AACUAUUCUA GGGCGCUGUG GCGGGUGGCU GCUGAGGAGU ACGUGGAGGU UACGCGGGUG 240
GGGGACUUCC ACUACGUGAC GGGCAUGACC ACUGACAACA UAAAGUGCCC AUGCCAGGUU 300
CCGGCCCCCG AGUUCUUCAC GGAGGUGGAU GGAGUGAGGU UGCACAGGUA CGCUCCGGCA 360
UGCAAACCUC UCCUACGGGA UGAGGUCACA UUCCAGGUCG GGCUCAACCA GUACCUGGUA 420
GGGUCACAGC UCCCAUGUGA GCCCGAACCG GACGUAGCUG UGCUUACUUC CAUGCUCACC 480
GAUCCCUCCC ACAUUACAGC AGAGACGGCC AAGCGUAGGC UGGCCAGGGG GUCUCCCCCC 540
UCCUUGGCCA GCUCUUCAGC UAGCCAGUUG UCUGCGCCUU CCUUGAAGGC GACAUGUACU 600
ACCCAUCAUG ACUCCCCGGA AGCUGACCUC AUCGAGGCCA ACCUCCUGUG GCGGCAGGAG 660
AUGGGCGGGA ACAUCACCCG CGUGGAGUCA GAAAAUAAGG UAGUAAUUCU GGACUCUUUC 720
GAUCCGCUCC GGGCGGAGGA GGAUGAGAGG GAAAUGUCCA UUCCGGCGGA GAUCCUGCGG 780
AAACCCAGGA GGUUCCCCCC AGCAUUGCCC AUAUGGGCAC GUGCGGAUUA CAACCCUCCA 840
CUGAUAGAAC CCUGGAAGGA CCCGGACUAC GUCCCUCCGG UGGUGCACGG GGAAUUC     897
```

SEQ ID NO: 20
SEQUENCE LENGTH: 218
SEQUENCE TYPE: nucleic acid
STRANDEDNESS: single
TOPOLOGY: linear
MOLECULE TYPE: Genomic RNA
ORIGINAL SOURCE: non-A, non-B hepatitis virus
SEQUENCE DESCRIPTION:

GAAUUCUUCU CUUGGGUGGA CGGGGUGCAA AUACACCGAU UCGCCCCCAC UCCAGGUCCC 60
UUCUUUCGGG AUGAGGUAAC GUUCACCGUG GGCCUCAAUU CCUUUGUGGU CGGCUCUCAG 120
CUCCCCUGCG ACCCUGAGCC GGACACCGAG GUAUUAGCCU CCAUGUUGAC AGACCCGUCC 180
CACAUCACCG CGGAGGCGGC AGCCAGGCGG UUGGCCAG 218

SEQ ID NO: 21
SEQUENCE LENGTH: 66
SEQUENCE TYPE: nucleic acid
STRANDEDNESS: single
TOPOLOGY: linear
MOLECULE TYPE: Genomic RNA
ORIGINAL SOURCE: non-A, non-B hepatitis virus
SEQUENCE DESCRIPTION:

GCCGCCCACA GGACGUCAAG UUCCCGGGCG GUGGUCAGAU CGUUGGUGGA GUUUACCUGU 60
UGCCGC 66

SEQ ID NO: 22
SEQUENCE LENGTH: 181
SEQUENCE TYPE: nucleic acid
STRANDEDNESS: single
TOPOLOGY: linear
MOLECULE TYPE: Genomic RNA
ORIGINAL SOURCE: non-A, non-B hepatitis virus
SEQUENCE DESCRIPTION:

CGUGCACCAU GAGCACAAAU CCUAAACCUC AAAGAAAAAC CAAAAGAAAU ACUAACCGUC 60
GCCCACAAGA CGUCAAGUUU CCGGGCGGCG GCCAGAUCGU UGGCGGAGUA UACUUGUUGC 120
CGCGCAGGGG CCCUAGGUUG GGUGUGCGCA CGACAAGGAA GACUUCGGAG CGAUCCCAGC 180
C 181

SEQ ID NO: 23
SEQUENCE LENGTH: 263
SEQUENCE TYPE: nucleic acid
STRANDEDNESS: single
TOPOLOGY: linear
MOLECULE TYPE: Genomic RNA
ORIGINAL SOURCE: non-A, non-B hepatitis virus
SEQUENCE DESCRIPTION:

CUCGUAGACC GUGCAUCAUG AGCACAAAUC CUAAACCUCA AAGAAAAACC AAAAGAAACA 60
CAAGCCGCCG CCCACAGGAC GUCAAGUUCC CGGGUGGCGG UCAGAUCGUU GGCGGAGUUU 120
ACUUGCUGCC GCGCAGGGGC CCCAGGUUGG GUGUGCGCGC GACAAGGAAG ACUUCCGAGC 180
GAUCCCAGCC GCGUGGGAGA CGCCAGCCCA UCCCUAAAGA UCGGCGCUCC ACCGGCAAGU 240
CCUGGGGAAA GCCAGGAUAU CCU 263

SEQ ID NO: 24
SEQUENCE LENGTH: 651
SEQUENCE TYPE: nucleic acid
STRANDEDNESS: single
TOPOLOGY: linear
MOLECULE TYPE: Genomic RNA
ORIGINAL SOURCE: non-A, non-B hepatitis virus
SEQUENCE DESCRIPTION:

```
CUACGGGCGC CCCCAUUACG UACUCCACCU AUGGUAAGUU CCUUGCCGAC GGUGGUUGCU    60
CUGGGGGCGC CUAUGACAUC AUAAUGUGCG AUGAGUGCCA CUCAACUGAC UCGACCUCCA   120
UCUUGGGCAU UGGCACGGUC CUGGACCAAG CGGAGACGGC UGGAGCGCGA CUUGUCGUGC   180
UCGCCACCGC UACCCCUCCG GGAUCGGUCA CUGUACCACA UCCCAAUAUC GAGGAGGUGG   240
CCCUGUCCAA CACUGGAGAG AUUCCCUUCU AUGGCAAAGC CAUCCCUAUC GAGACCAUCA   300
AGGGGGGGAG GCAUCUCAUU UUUUGCCACU CUAAGAAGAA GUGUGAUGAG CUCGCCACAA   360
AGCUGUCGGC CCUCGGACUC AAUGCUGUAG CGUACUACCG GGGCCUUGAU GUGUCCGUUA   420
UACCAACAAG CGGAGACGUC GUUGUCGUGG CAACAGACGC UCUAAUGACG GGCUACACCG   480
GUGACUUUGA CUCAGUGAUC GACUGUAAUA CAUGCGUCAC CCAGACAGUC GAUUUCAGCU   540
UGGACCCUAC CUUCACCAUU GAGACGACAA CCGUGCCUCA AGACGCGGUG UCACGCUCGC   600
AGCGGCGAGG UAGGACUGGU AGGGGCAGGG GGGGCAUAUA CAGGUUUGUA G            651
```

## Claims

1. An antigenic protein related to non-A, non-B hepatitis virus comprising at least one of the amino acid sequences shown by SEQ ID NOs. 1 - 12 in Sequence Listing or an antigenic protein substantially equivalent thereto.

2. The antigenic protein according to claim (1) which is an antigenic protein related to non-A, non-B hepatitis virus comprising the amino acid sequence shown by SEQ ID NO. 1 in Sequence Listing.

3. The antigenic protein according to claim (1) which is an antigenic protein related to non-A, non-B hepatitis virus comprising the amino acid sequence shown by SEQ ID NO. 2 in Sequence Listing.

4. The antigenic protein according to claim (1) which is an antigenic protein related to non-A, non-B hepatitis virus comprising the amino acid sequence shown by SEQ ID NO. 3 in Sequence Listing.

5. The antigenic protein according to claim (1) which is an antigenic protein related to non-A, non-B hepatitis virus comprising the amino acid sequence shown by SEQ ID NO. 4 in Sequence Listing.

6. The antigenic protein according to claim (1) which is an antigenic protein related to non-A, non-B hepatitis virus comprising the amino acid sequence shown by SEQ ID NO. 5 in Sequence Listing.

7. The antigenic protein according to claim (1) which is an antigenic protein related to non-A, non-B hepatitis virus comprising the amino acid sequence shown by SEQ ID NO. 6 in Sequence Listing.

8. The antigenic protein according to claim (1) which is an antigenic protein related to non-A, non-B hepatitis virus comprising the amino acid sequence shown by SEQ ID NO. 7 in Sequence Listing.

9. The antigenic protein according to claim (1) which is an antigenic protein related to non-A, non-B hepatitis virus comprising the amino acid sequence shown by SEQ ID NO. 8 in Sequence Listing.

10. The antigenic protein according to claim (1) which is an antigenic protein related to non-A, non-B hepatitis virus comprising the amino acid sequence shown by SEQ ID NO. 9 in Sequence Listing.

11. The antigenic protein according to claim (1) which is an antigenic protein related to non-A, non-B hepatitis virus comprising the amino acid sequence shown by SEQ ID NO. 10 in Sequence Listing.

12. The antigenic protein according to claim (1) which is an antigenic protein related to non-A, non-B hepatitis virus comprising the amino acid sequence shown by SEQ ID NO. 11 in Sequence Listing.

13. The antigenic protein according to claim (1) which is an antigenic protein related to non-A, non-B hepatitis virus comprising the amino acid sequence shown by SEQ ID NO. 12 in Sequence Listing.

14. The antigenic protein according to claim (1) wherein the antigenic protein substantially equivalent to the antigenic protein related to non-A, non-B hepatitis virus is selected from T064, T069 and T06A.

15. A DNA coding for the antigenic protein according to claim (1) or an antigenic protein substantially equivalent thereto.

16. A DNA coding for the antigenic protein according to claim (2).

17. A DNA coding for the antigenic protein according to claim (3).

18. A DNA coding for the antigenic protein according to claim (7).

19. A non-A, non-B hepatitis virus genome RNA which has at least one of the base sequences shown by SEQ ID NOs. 13 - 24 in Sequence Listing or the base sequences substantially derived therefrom.

20. An agent for detecting non-A, non-B hepatitis comprising as the principal component at least one of the amino acid sequences shown by SEQ ID NOs. 1 - 12 in Sequence Listing or a substantially equivalent sequence thereto.

21. The agent for detecting non-A, non-B hepatitis according to claim (20) which contains as the principal component an antigenic protein comprising the amino acid sequence shown by SEQ ID NO. 1 in Sequence Listing or a substantially equivalent sequence thereto.

22. The agent for detecting non-A, non-B hepatitis according to claim (20) which contains as the principal component an antigenic protein comprising the amino acid sequence shown by SEQ ID NO. 2 in Sequence Listing or a substantially equivalent sequence thereto.

23. The agent for detecting non-A, non-B hepatitis according to claim (20) which contains as the principal component an antigenic protein comprising the amino acid sequence shown by SEQ ID NO. 6 in Sequence Listing or a substantially equivalent sequence thereto.

24. The agent for detecting non-A, non-B hepatitis according to claim (20) wherein the antigenic protein substantially equivalent to the antigenic protein related to non-A, non-B hepatitis virus is selected from T064, T069 and T06A.

25. A agent for detecting non-A, non-B hepatitis comprising a combination of at least two of the antigenic proteins according to claim (1) or substantial equivalents thereto.

26. The agent for detecting non-A, non-B hepatitis according to claim (25) comprising a combination of the antigenic proteins according to claim (2) or a substantially equivalent sequence thereto and the antigenic proteins according to claim (3) or a substantially equivalent sequence thereto.

**27.** An expression vector which is ligated downstream the promoter capable of expressing cDNA coding for an antigenic protein related to non-A, non-B hepatitis virus in a host and can be expressed in the host.

**28.** The expression vector according to claim (25) wherein said cDNA is cDNA coding for an antigenic protein comprising at least one of the amino acid sequences shown by SEQ ID NOs. 1 - 12 in Sequence Listing or an antigenic protein substantially equivalent thereto.

**29.** A transformant possessing the expression vector according to claim (27).

**30.** A transformant possessing the expression vector according to claim (28).

**31.** The transformant according to claim (27) produced using E. coli as the host.

**32.** A method for preparing antigenic proteins related to non-A, non-B hepatitis virus which comprises culturing the transformant according to claim (29).

**33.** The method for preparing the antigenic proteins according to claim (32) which is obtained as a fused protein.

MSTNPKPQRK TKRNTSRRPQ DVKFPGGGQI
T064
VGGVYLLPRR GPRLGVRATR KTSERSQPRG
T069
RRQPIPKDRR STGKSWGKPG YP

T06A

FIG. 1 RELATIONSHIP BETWEEN AMINO ACID SEQUENCE OF S29 AND DESIGN OF SYNTHETIC PEPTIDES

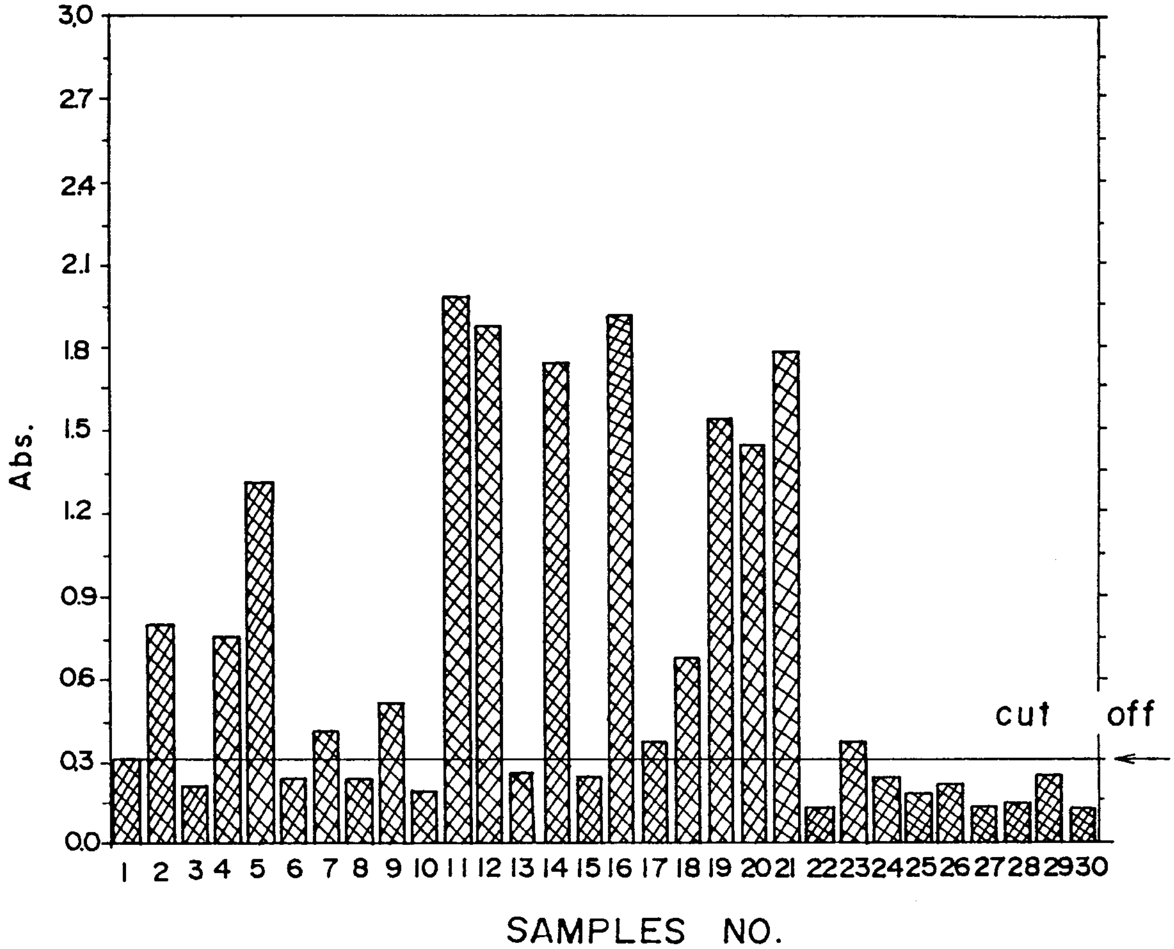


ELISA USING TO6A AS ANTIGEN

FIG. 2

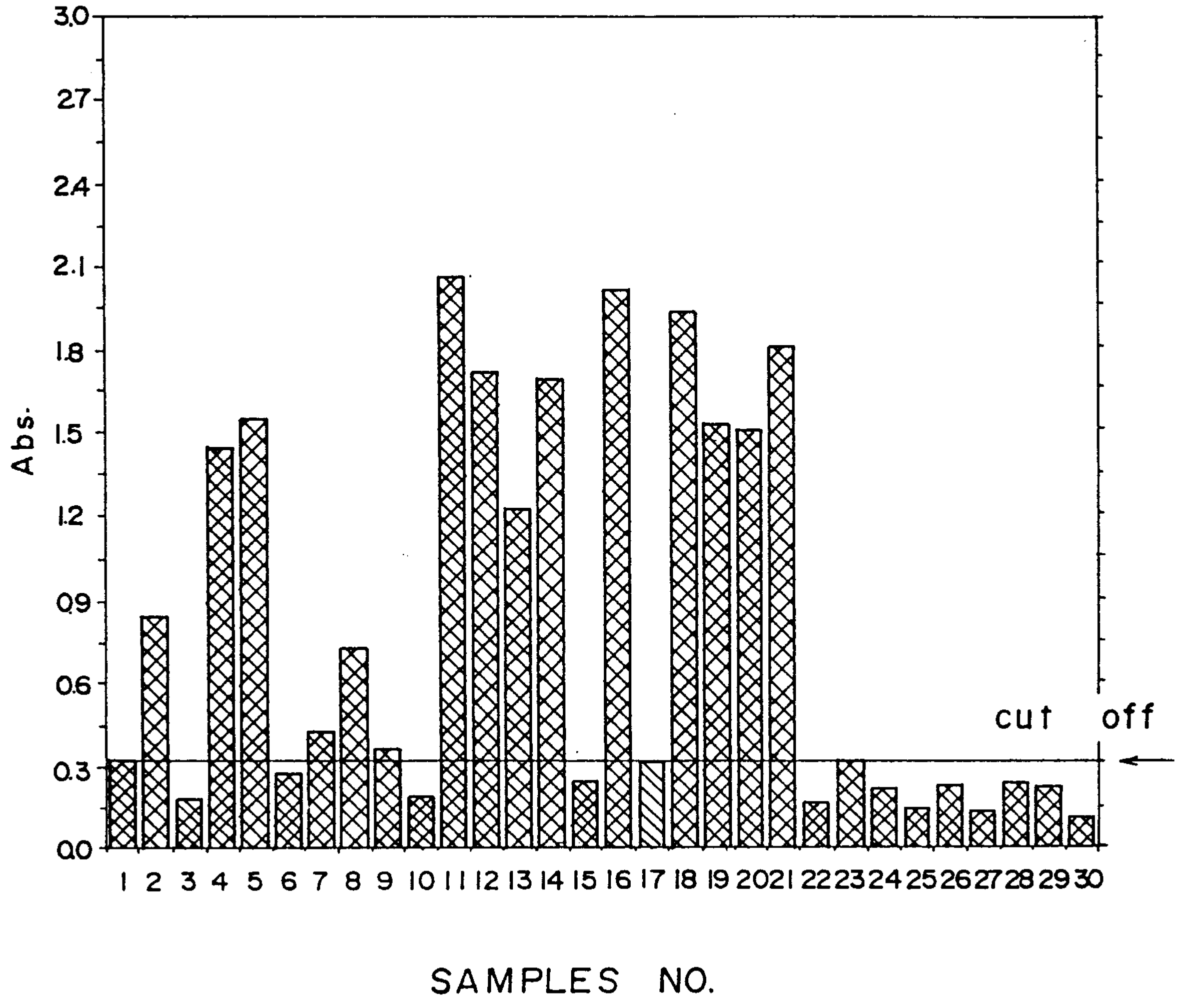


ELISA USING TO69 AS ANTIGEN

FIG. 3

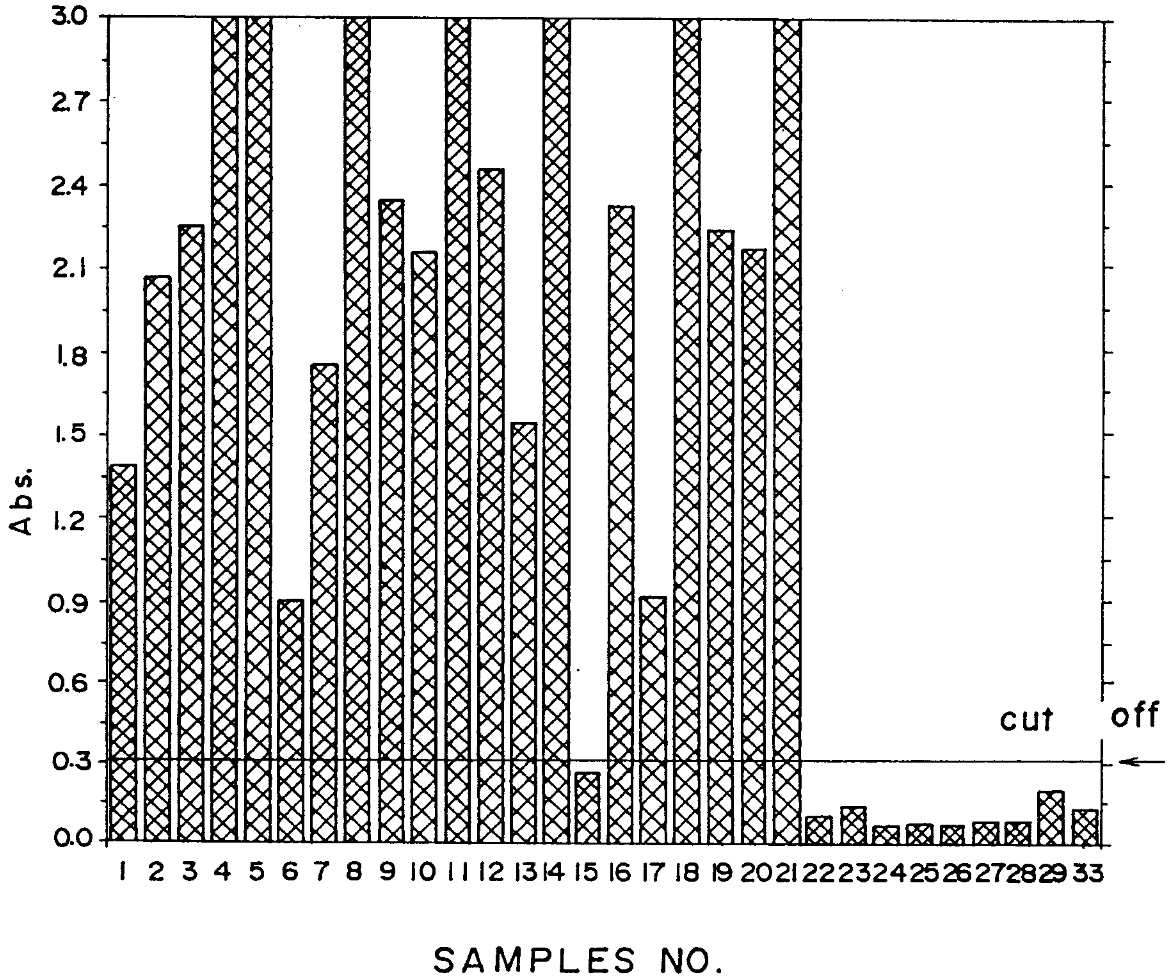


ELISA USING TO69 AS ANTIGEN

FIG. 4

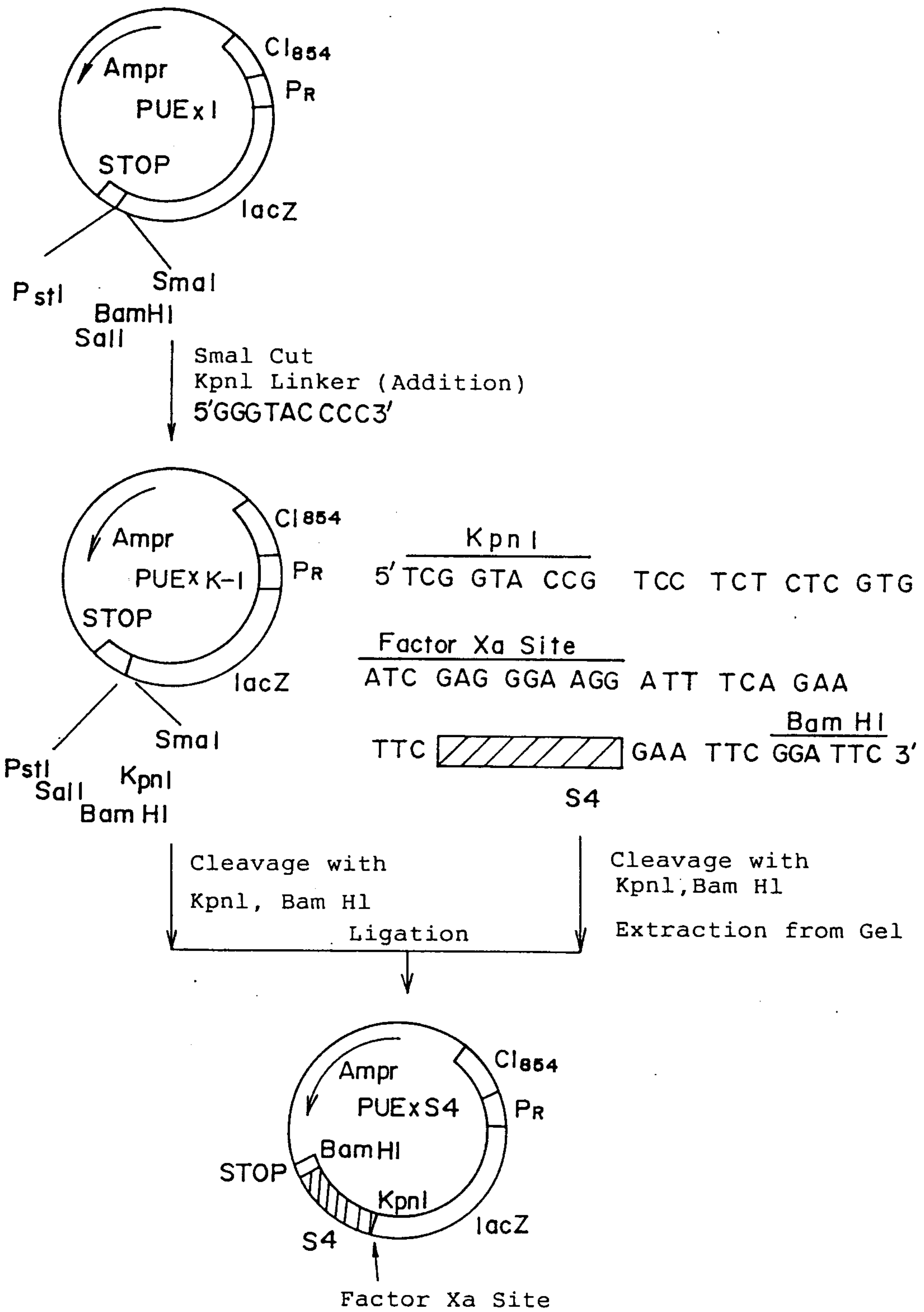
Ampr
PUEx I
CI854
PR
STOP
lacZ
PstI
SmaI
BamHI
SalI
SmaI Cut
KpnI Linker (Addition)
5'GGGTACCCC3'
Ampr
PUEx K-I
CI854
PR
STOP
lacZ
PstI
SalI
SmaI
KpnI
Bam HI
Kpn I
5' TCG GTA CCG TCC TCT CTC GTG
Factor Xa Site
ATC GAG GGA AGG ATT TCA GAA
Bam HI
TTC GAA TTC GGA TTC 3'
S4
Cleavage with
KpnI, Bam HI
Ligation
Cleavage with
KpnI, Bam HI
Extraction from Gel
Ampr
PUExS4
CI854
PR
BamHI
STOP
KpnI
S4
lacZ
Factor Xa Site

FIG. 5

ELISA ON MICROPLATE

(SYNTHETIC PEPTIDE TO64+RECOMBINANT ANTIGEN S4)

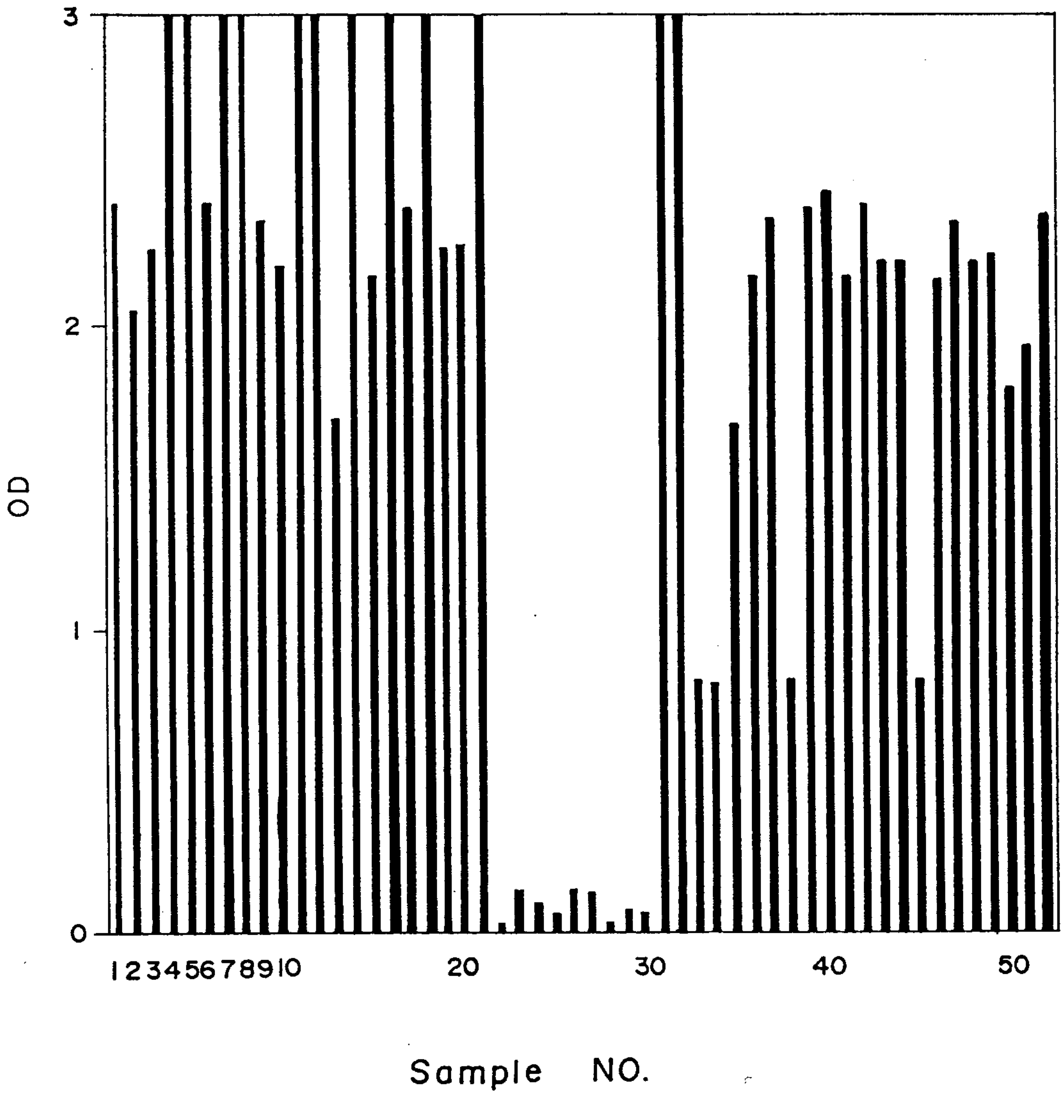


FIG. 6

ELISA ON MICROPLATE

(SYNTHETIC PEPTIDE TO64+RECOMBINANT ANTIGEN S4)

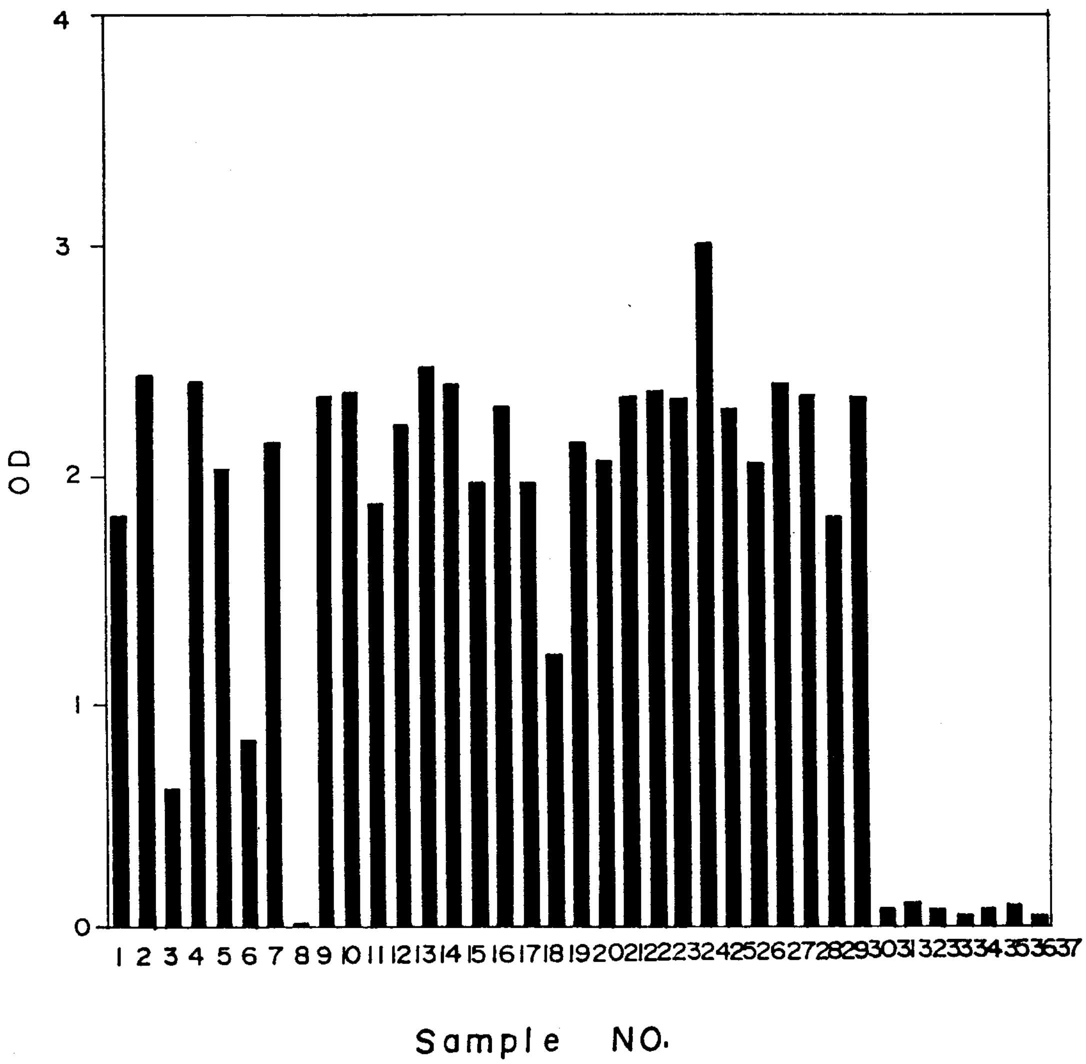


FIG. 7

FIG. 8

REACTIVITY AGAINST ANTIGENS S4 AND S29
(TO64)

| SAMPLE | T064 | S4 | SAMPLE | T064 | S4 |
|---|---|---|---|---|---|
| 1 | + | + | 16 | − | + |
| 2 | + | + | 17 | − | + |
| 3 | + | − | 18 | + | − |
| 4 | + | + | 19 | + | + |
| 5 | + | + | 20 | + | + |
| 6 | − | + | 21 | + | + |
| 7 | + | + | 22 | + | + |
| 8 | − | − | 23 | + | + |
| 9 | + | − | 24 | + | + |
| 10 | + | + | 25 | + | + |
| 11 | + | + | 26 | + | + |
| 12 | + | + | 27 | + | + |
| 13 | + | + | 28 | + | + |
| 14 | + | + | 29 | + | − |
| 15 | + | + | 30 | + | + |

# INTERNATIONAL SEARCH REPORT

International Application No PCT/JP91/01662

**I. CLASSIFICATION OF SUBJECT MATTER** (if several classification symbols apply, indicate all) [6]

According to International Patent Classification (IPC) or to both National Classification and IPC Int. $Cl^5$

C07K15/04, C12P21/02, C12N1/21, 15/51, 15/62, 15/70, G01N33/576//(C12P21/02, C12R1:19) (C12N1/21, C12R1:19)

**II. FIELDS SEARCHED**

Minimum Documentation Searched [7]

| Classification System | Classification Symbols |
|---|---|
| IPC | C07K15/04, C12P21/02, C12N1/21, 15/51, 15/62, G01N33/576 |

Documentation Searched other than Minimum Documentation to the Extent that such Documents are Included in the Fields Searched [8]

Chemical Abstracts Data Base (REGISTRY, CA)

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| X/A | JP, A, 2-500880 (Chiron Corporation), March 29, 1990 (29. 03. 90) & EP, A1, 318216 & WO, A1, 89/4669 | 1, 15, 20, 25-33/2-14, 16-19, 21-24 |
| X/A | EP, A1, 388232 (Chiron Corporation), September 19, 1990 (19. 09. 90) & WO, A1, 90/11089 | 1, 15, 20, 25-33/2-14, 16-19, 21-24 |
| P,X/A | WO, A1, 90/14436 (Chiron Corporation), November 29, 1990 (29. 11. 90) & EP, A2, 398748 & CA, A, 2017157 | 1, 15, 20, 25-33/2-14, 16-19, 21-24 |
| P,X/A | WO, A1, 91/4262 (National Institute of Health of Japan), April 4, 1991 (04. 04. 91) & EP, A1, 419182 | 1, 15, 20, 25-33/2-14, 16-19, 21-24 |
| P,X/A | GB, A, 2239245 (Wellcome Foundation Ltd.), June 26, 1991 (26. 06. 91) & DE, A1, 4040339 & FR, A1, 2655990 & CA, A, 2032381 | 1, 15, 20, 25-33/2-14, 16-19, 21-24 |
| P,X/A | EP, A2, 442394 (United Biomedical, Inc.), | 1, 20/2-19, |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| February 19, 1992 (19. 02. 92) | March 10, 1992 (10. 03. 92) |
| **International Searching Authority** | **Signature of Authorized Officer** |
| Japanese Patent Office | |

International Application No. PCT/JP91/01662

FURTHER INFORMATION CONTINUED FROM THE SECOND SHEET

| | | |
|---|---|---|
| | August 21, 1991 (21. 08. 91) & CA, A, 2036463 | 21-33 |
| P,X/A | EP, A2, 445423 (Abbott Laboratories), September 11, 1991 (11. 09. 91) & CA, A, 2032907 | 1, 15, 20, 25-33/2-14, 16-19, 21-24 |
| P,X/A | Proceedings of the National Academy of Sciences, U.S.A., Vol. 87, No. 24, (1990), N. Kato et al. "Molecular cloning of the human hepatitis C virus genome from Japanese patients with non-A, non-B hepatitis" p. 9524-9528 | 1, 15/2-14, 16-33 |

V. ☐ OBSERVATIONS WHERE CERTAIN CLAIMS WERE FOUND UNSEARCHABLE [1]

This international search report has not been established in respect of certain claims under Article 17(2) (a) for the following reasons:

1. ☐ Claim numbers , because they relate to subject matter not required to be searched by this Authority, namely:

2. ☐ Claim numbers , because they relate to parts of the international application that do not comply with the prescribed requirements to such an extent that no meaningful international search can be carried out, specifically:

3. ☐ Claim numbers , because they are dependent claims and are not drafted in accordance with the second and third sentences of PCT Rule 6.4(a).

VI. ☐ OBSERVATIONS WHERE UNITY OF INVENTION IS LACKING [2]

This International Searching Authority found multiple inventions in this international application as follows:

1. ☐ As all required additional search fees were timely paid by the applicant, this international search report covers all searchable claims of the international application.

2. ☐ As only some of the required additional search fees were timely paid by the applicant, this international search report covers only those claims of the international application for which fees were paid, specifically claims:

3. ☐ No required additional search fees were timely paid by the applicant. Consequently, this international search report is restricted to the invention first mentioned in the claims; it is covered by claim numbers:

4. ☐ As all searchable claims could be searched without effort justifying an additional fee, the International Searching Authority did not invite payment of any additional fee.

Remark on Protest

☐ The additional search fees were accompanied by applicant's protest.

☐ No protest accompanied the payment of additional search fees.

**FURTHER INFORMATION CONTINUED FROM THE SECOND SHEET**

| | | |
|---|---|---|
| P,X/A | Journal of Virology, Vol. 65, No. 3, (1991), A. Takamizawa et al. "Structure and organization of the hepatitis C virus genome isolated from human carriers" p. 1105-1113 | 1, 15/2-14, 16-33 |
| P,X/A | Proceedings of the National Academy of Sciences, U.S.A., Vol. 88, No. 6, (1991), Q.-L. Choo et al. "Genetic organization and diversity of the hepatitis C virus" p. 2451-2455 | 1, 15/2-14, 16-33 |

**V. ☐ OBSERVATIONS WHERE CERTAIN CLAIMS WERE FOUND UNSEARCHABLE [1]**

This international search report has not been established in respect of certain claims under Article 17(2) (a) for the following reasons:

1. ☐ Claim numbers , because they relate to subject matter not required to be searched by this Authority, namely:

2. ☐ Claim numbers , because they relate to parts of the international application that do not comply with the prescribed requirements to such an extent that no meaningful international search can be carried out, specifically:

3. ☐ Claim numbers , because they are dependent claims and are not drafted in accordance with the second and third sentences of PCT Rule 6.4(a).

**VI. ☐ OBSERVATIONS WHERE UNITY OF INVENTION IS LACKING [2]**

This International Searching Authority found multiple inventions in this international application as follows:

1. ☐ As all required additional search fees were timely paid by the applicant, this international search report covers all searchable claims of the international application.

2. ☐ As only some of the required additional search fees were timely paid by the applicant, this international search report covers only those claims of the international application for which fees were paid, specifically claims:

3. ☐ No required additional search fees were timely paid by the applicant. Consequently, this international search report is restricted to the invention first mentioned in the claims; it is covered by claim numbers:

4. ☐ As all searchable claims could be searched without effort justifying an additional fee, the International Searching Authority did not invite payment of any additional fee.

**Remark on Protest**

☐ The additional search fees were accompanied by applicant's protest.

☐ No protest accompanied the payment of additional search fees.